# EUROPÄISCHE PATENTSCHRIFT

(11) **EP 0 713 529 B1**
(45) Veröffentlichungstag und Bekanntmachung des Hinweises auf die Patenterteilung: **02.02.2000**
(21) Anmeldenummer: 94924856.1
(22) Anmeldetag: 09.08.1994
(51) Int. Cl.: C12N 15/12, C07K 14/51, C07K 14/495, A61K 38/18, C07K 16/22

(54) **Wachstums-Differenzierungsfaktor der TGF-B Familie**
Growth differentiation factor of the TGF-B family
Facteur de croissance et de differenciation de la famille de TGF-B

(30) Priorität: 10.08.1993 DE 4326829; 25.05.1994 DE 4418222; 09.06.1994 DE 4420157
(43) Veröffentlichungstag der Anmeldung: 29.05.1996
(73) Patentinhaber: BIOPHARM GESELLSCHAFT ZUR BIOTECHNOLOGISCHEN ENTWICKLUNG VON PHARMAKA mbH, 69115 Heidelberg (DE)
(72) Erfinder: HÖTTEN, Gertrud, 44625 Herne (DE); NEIDHARDT, Helge, D-35041 Marburg (DE); PAULISTA, Michael, D-69181 Leimen (DE)
(74) Vertreter: Böhm, Brigitte, Dipl.-Chem. Dr.
(86) Internationale Anmeldenummer: EP9402630
(87) Internationale Veröffentlichungsnummer: WO9504819

(56) Entgegenhaltungen:
- WO-A-90/11366
- WO-A-91/05802
- WO-A-93/16099

## Beschreibung

Die vorliegende Erfindung betrifft einen neuen Wachstums/Differenzierungsfaktor der TGF-β-Familie und dafür codierende DNA-Sequenzen.

Die TGF-β-Familie von Wachstumsfaktoren, zu der BMP-, TGF- und Inhibin-verwandte Proteine gehören (Roberts und Sporn, Handbook of Experimental Pharmacology 95 (1990), 419-472) ist besonders für einen weiten Bereich medizinischer Behandlungsmethoden und Anwendungen relevant. Diese Faktoren eignen sich in Verfahren, welche die Wundheilung und die Gewebewiederherstellung betreffen. Weiterhin induzieren mehrere Mitglieder der TGF-β-Familie das Gewebewachstum, insbesondere das Wachstum von Knochen und spielen daher eine zentrale Rolle bei der Induzierung der Entwicklung von Knorpeln und Knochen.

Wozney (Progress in Growth Factor Research 1 (1989), 267-280) und Vale et al. (Handbook of Experimental Pharmacology 95 (1990), 211-248) beschreiben verschiedene Wachstumsfaktoren, wie etwa diejenigen, die mit der BMP-(knochenmorphogenetische Proteine) und der Inhibin-Gruppe verwandt sind. Die Mitglieder dieser Gruppen weisen signifikante strukturelle Ähnlichkeiten auf. Der Vorläufer des Proteins besteht aus einer aminoterminalen Signalsequenz, einer Propeptid- und einer carboxyterminalen Sequenz von etwa 110 Aminosäuren, die vom Vorläufer abgespalten wird und das reife Protein darstellt. Weiterhin sind ihre Mitglieder durch eine Aminosäuresequenzhomologie definiert. Das reife Protein enthält die am höchsten konservierten Sequenzen, insbesondere sieben Cysteinreste, die unter den Familienmitgliedern konserviert sind. Die TGF-β-artigen Proteine sind multifunktionelle, hormonell aktive Wachstumsfaktoren. Sie weisen auch verwandte biologische Aktivitäten, wie etwa chemotaktische Attraktion von Zellen, Förderung der Zelldifferenzierung und Gewebe-induzierende Fähigkeiten, wie etwa Knorpel-, Knochen-induzierende Fähigkeiten auf. Das US-Patent Nr. 5,013,649 offenbart DNA-Sequenzen, die für osteoinduktive, als BMP-2 bezeichnete Proteine codieren, und die US-Patentanmeldungen Serien Nr. 179 101 und 170 197 offenbaren die BMP-Proteine BMP-1 und BMP-3. Die WO90/11366 beschreibt die osteoinduktiven Proteine BMP-5, BMP-6 und BMP-7. Diese Proteine können zur Behandlung von Knochen- und Knorpeldefekten ebenso eingesetzt werden wie zur Wundbehandlung. Weiterhin sind viele Zelltypen zur Synthese TGF-β-artiger Proteine in der Lage, und praktisch alle Zellen besitzen TGF-β-Rezeptoren.

Insgesamt zeigen diese Proteine Unterschiede in ihrer Struktur, was zu erheblichen Variationen in ihrer genauen biologischen Funktion führt. Weiterhin werden sie in einem weiten Bereich unterschiedlicher Gewbearten und Entwicklungsstufen gefunden. Folglich können sie Unterschiede hinsichtlich ihrer genauen Funktion, z.B. der erforderlichen zellulären physiologischen Umgebung, ihrer Lebensdauer, ihrer Zielorte, ihrer Erfordernisse für Hilfsfaktoren und ihrer Beständigkeit gegen Abbau aufweisen. Obwohl daher zahlreiche Proteine, die ein Gewebe-induktives, insbesondere ein osteo-induktives Potential zeigen, beschrieben werden, müssen ihre natürlichen Aufgaben im Organismus und - noch bedeutsamer - ihre medizinische Relevanz im Detail noch erforscht werden. Das Vorhandensein von noch unbekannten Mitgliedern der TGF-β-Familie, die für die Osteogenese oder die Differenzierung/Induktion von anderen Gewebearten bedeutsam sind, wird mit großer Wahrscheinlichkeit angenommen. Eine große Schwierigkeit bei der Isolierung dieser neuen TGF-β-artigen Proteine besteht jedoch darin, daß ihre Funktionen noch nicht genau genug für die Entwicklung eines unterscheidungskräftigen Bioassays beschrieben werden können. Andererseits ist die erwartete Nukleotidsequenzhomologie zu bekannten Mitgliedern der Familie zu gering, um ein Screening durch klassiche Nukleinsäurehybridisierungstechniken zu ermöglichen. Dennoch ist die weitere Isolierung und Charakterisierung von neuen TGF-β-artigen Proteinen dringend erforderlich, um weitere Induzierungs- und Differenzierungsproteine bereitzustellen, die alle gewünschten medizinischen Erfordernisse erfüllen. Diese Faktoren könnten medizinische Anwendung bei der Heilung von Schäden und der Behandlung von degenerativen Erkrankungen der Knochen und/oder anderen Gewebearten, wie etwa z.B. Niere oder Leber, finden.

In der Patentanmeldung WO93/16099 ist eine Nukleotid- und Aminosäuresequenz für das TGF-β-Protein MP-52 angegeben, wobei die dem reifen Peptid entsprechende Sequenz und ein Großteil der dem Propeptid von MP-52 entsprechenden Sequenz angegeben ist. Die vollständige Sequenz des Propeptids MP-52 wird nicht offenbart.

Die der vorliegenden Erfindung zugrundeliegende Aufgabe besteht darin, DNA-Sequenzen bereitzustellen, die für neue Mitglieder der TGF-β-Proteinfamilie mit mitogenem und/oder differenzierungsinduktivem, z.B. osteo-induktivem, Potential codieren. Insbesondere besteht die Aufgabe der vorliegenden Erfindung darin, die vollständige DNA- und Aminosäuresequenz des TGF-Proteins MP-52 bereitzustellen.

Diese Aufgabe wird gelöst durch ein DNA-Molekül, welches für ein Protein der TGF-β-Familie codiert und aus einer der folgenden DNA-Sequenzen besteht:
a) Nukleotiden 1783 - 2142 oder 640 bis 2142 der SEQ ID NO.1,
b) einer der Sequenz aus a) im Rahmen der Degeneration des genetischen Codes entsprechende Nukleotidsequenz,
c) einem allelischen Derivat einer der Sequenzen aus a) und b) entsprechende Nukleotidsequenz, oder
d) einer mit einer der Sequenzen aus a), b) oder c) bei stringenten Hybridisierungsbedingungen hybridisierende Nukleotidsequenz unter der Voraussetzung, dass ein DNA-Molekül gemäß d) zumindest den für ein reifes Protein der TGF-β-Familie codierenden Anteil vollständig enthält, und die Nukleotidsequenz nicht zu SEQ ID NO.1 vollständig komplementär ist.

Weitere Ausführungsformen der vorliegenden Erfindung betreffen den Gegenstand der Ansprüche 2 bis 12. Andere Merkmale und Vorteile der Erfindung gehen aus der Beschreibung der bevorzugten Ausführungsformen und den Zeichnungen hervor. Die Sequenzprotokolle und Zeichnungen werden jetzt kurz beschrieben.

SEQ ID NO.1 zeigt die vollständige Nukleotidsequenz der für das TGF-β-Protein MP-52 codierenden DNA. Das ATG-Startcodon beginnt mit Nukleotid 640. Der Start des reifen Proteins beginnt hinter Nukleotid 1782.

SEQ ID NO.2 zeigt die vollständige Aminosäuresequenz des TGF-β-Proteins MP-52, die aus der in SEQ ID NO.1 gezeigten Nukleotidsequenz abgeleitet wurde.

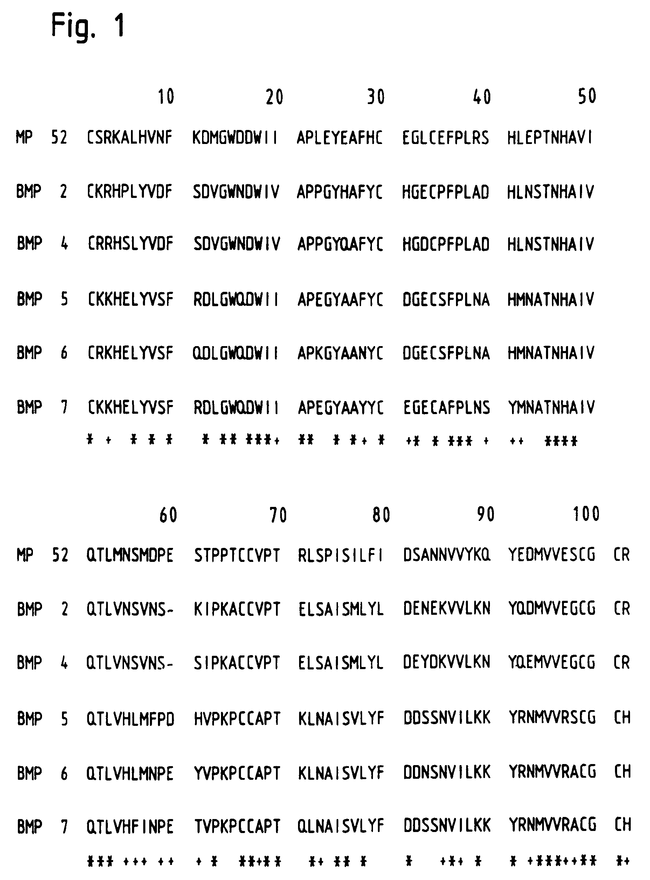

Figur 1 zeigt einen Vergleich der Aminosäuresequenz von MP-52 mit einigen Mitgliedern der BMP-Proteinfamilie mit Beginn am ersten der sieben konservierten Cysteinreste. * bedeutet, daß die Aminosäure in allen verglichenen Proteinen gleich ist; + bedeutet, daß die Aminosäure in mindestens einem der Proteine im Vergleich zu MP-52 übereinstimmt.

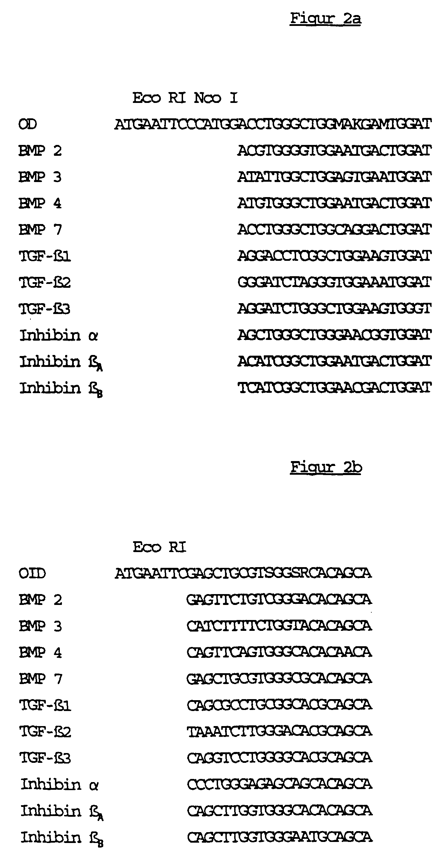

Figur 2 zeigt die Nukleotidsequenzen der Oligonukleotidprimer, die in der vorliegenden Erfindung verwendet wurden, und einen Vergleich dieser Sequenzen mit bekannten Mitgliedern der TGF-β-Familie. M bedeutet A oder C, S bedeutet C oder G, R bedeutet A oder G und K bedeutet G oder T. 2a zeigt die Sequenz des Primers OD. 2b zeigt die Sequenz des Primers OID.

Die vorliegende Erfindung umfaßt zumindest den für das reife Protein codierenden Anteil und gegebenenfalls weitere funktionelle Anteile der in SEQ ID NO.1 gezeigten Nukleotidsequenz sowie Sequenzen, die dieser Sequenz im Rahmen der Degeneration des genetischen Codes entsprechen und allelische Derivate solcher Sequenzen. Weiterhin umfaßt die vorliegende Erfindung auch mit derartigen Sequenzen hybridisierende Sequenzen unter der Voraussetzung, daß ein solches DNA-Molekül zumindest den für ein reifes Protein der TGF-β-Familie codierenden Anteil vollständig enthält.

Der Begriff "funktioneller Anteil" im Sinne der vorliegenden Erfindung bedeutet einen Proteinanteil, der in der Lage ist, z.B. als Signalpeptid-, Propeptid- bzw. reifer Proteinanteil zu wirken, d.h. mindestens eine der biologischen Funktionen der natürlichen Proteinanteile von MP-52 zu erfüllen.

Der für den reifen Anteil des Proteins codierende Bereich reicht von den Nukleotiden 1783 - 2142 der in SEQ ID NO. 1 gezeigten Sequenz. Gegebenenfalls kann das DNA-Molekül noch weitere funktionelle Anteile der in SEQ ID NO. 1 gezeigten Sequenz umfassen, nämlich die für den Signal- oder/und Propeptidanteil codierenden Nukleotidsequenzen. Besonders bevorzugt umfaßt das DNA-Molekül die Sequenz für den Signal- und den Propeptidanteil und den Anteil des reifen Proteins, d.h. die Nukleotide 640-2142 der in SEQ ID NO.1 gezeigten Sequenz. Andererseits kann das DNA-Molekül neben dem für das reife Protein codierenden Anteil auch noch funktionelle Signal- oder/und Propeptidanteile von anderen Proteinen, insbesondere von anderen Proteinen der TGF-β-Familie, z.B. den oben genannten BMP-Proteinen, umfassen. Die entsprechenden Nukleotidsequenzen sind aus den oben genannten Referenzen zu entnehmen, auf deren Offenbarung hiermit Bezug genommen wird.

Weiterhin umfaßt die vorliegende Erfindung auch ein DNA-Molekül wie oben definiert, das zusätzlich zwischen den Nukleotiden 1270 und 1271 der in SEQ ID NO. 1 gezeigten Sequenz eine nicht-codierende Intronsequenz enthält. Diese Intronsequenz ist in dem bei DSM hinterlegten Plasmid SKL 52 (H3) MP12 enthalten, das die genomische Nukleinsäuresequenz von MP-52 aufweist.

Auch von der Erfindung umfaßt ist die vom Phagen λ 15.1 codierte cDNA-Sequenz des MP-52 Proteins. Diese Sequenz beginnt mit Nukleotid 321 von SEQ ID NO. 1.

Obwohl die allelischen, degenerierten und hybridisierenden Sequenzen, die von der vorliegenden Erfindung umfaßt werden, strukturelle Unterschiede aufgrund geringfügiger Änderungen in der Nukleotid- oder/und Aminosäuresequenz aufweisen, besitzen die von derartigen Sequenzen codierten Proteine noch im wesentlichen die gleichen nützlichen Eigenschaften, die ihren Einsatz in grundsätzlich den gleichen medizinischen Anwendungen ermöglichen.

Gemäß vorliegender Erfindung bedeutet die Bezeichnung "Hybridisierung" übliche Hybridisierungsbedingungen, vorzugsweise Bedingungen mit einer Salzkonzentration von 6 x SSC bei 62 bis 66°C, gefolgt von einem einstündigen Waschen mit 0,6 x SSC, 0,1 % SDS bei 62° bis 66°C. Besonders bevorzugt betrifft die Bezeichnung "Hybridisierung" stringente Hybridisierungsbedingungen mit einer Salzkonzentration von 4 x SSC bei 62 bis 66°C, gefolgt von einem einstündigen Waschen mit 0,1 x SSC, 0,1 % SDS bei 62 bis 66°C.

Bevorzugte Ausführungsformen der vorliegenden Erfindung sind DNA-Sequenzen, wie oben definiert, die aus Wirbeltieren, vorzugsweise Säugern, wie etwa Schweinen, Kühen und Nagern, wie etwa Ratten oder Mäusen, und insbesondere von Primaten, wie etwa Menschen, erhältlich sind.

Eine besonders bevorzugte Ausführungsform der vorliegenden Erfindung ist die in SEQ ID NO. 1 gezeigte und als MP-52 bezeichnete Sequenz. Die Transkripte von MP-52 wurden aus embryonalem Gewebe erhalten und codieren für ein Protein, das eine beträchtliche Aminosäurenhomologie zum reifen Teil der BMP-artigen Proteine zeigt (siehe Fig. 1). Die Proteinsequenzen von BMP2 (= BMP2A) und BMP4 (= BMP2B) sind bei Wozney et al., Science 242 (1988), 1528-1534 beschrieben. Die entsprechenden Sequenzen von BMP5, BMP6 und BMP7 sind bei Celeste et al., Proc. Natl. Acad. Sci. USA 87 (1990), 9843-9847 beschrieben. Einige typische Sequenzhomologien, die für bekannte BMP-Sequenzen spezifisch sind, wurden auch im Propeptidteil von MP-52 gefunden, während andere Teile des Precursorteils von MP-52 erhebliche Unterschiede zu BMP-Precursoren zeigen.

Ein weiterer Gegenstand der vorliegenden Erfindung ist ein Vektor, der mindestens eine Kopie eines erfindungsgemäßen DNA-Moleküls enthält. In einem derartigen Vektor ist die erfindungsgemäße DNA-Sequenz vorzugsweise operativ mit einer Expressionskontrollsequenz verknüpft. Solche Vektoren eignen sich zur Herstellung von TGF-β-artigen Proteinen in stabil- oder transient-transformierten Zellen. Verschiedene Tier-, Pflanzen-, Pilz- und Bakteriensysteme können zur Transformation und die anschließende Kultivierung verwendet werden. Vorzugsweise enthalten die erfindungsgemäßen Vektoren für die Replikation in der Wirtszelle notwendige Sequenzen und sind autonom replizierbar. Weiterhin ist die Verwendung von Vektoren bevorzugt, die selektierbare Markergene enthalten, wodurch die Transformation einer Wirtszelle nachweisbar ist.

Ein weiterer Gegenstand der Erfindung ist eine Wirtszelle, die mit einer erfindungsgemäßen DNA oder einem erfindungsgemäßen Vektor transformiert ist. Beispiele von geeigneten Wirtszellen umfassen verschiedene eukaryontische und prokaryontische Zellen, wie etwa E.coli, Insektenzellen, Pflanzenzellen, Säugerzellen und Pilze, wie etwa Hefe.

Ein weiterer Gegenstand der Erfindung ist ein Protein der TGF-β-Familie, das von einer DNA-Sequenz nach Anspruch 1 codiert wird. Vorzugsweise weist das erfindungsgemäße Protein die in SEQ ID NO.2 gezeigte Aminosäuresequenz oder gegebenenfalls funktionelle Anteile davon auf und zeigt biologische Eigenschaften, wie etwa Gewebe-induktive, insbesondere osteoinduktive oder/und mitogene Fähigkeiten, die möglicherweise für eine therapeutische Anwendung relevant sind. Die oben genannten Merkmale des Proteins können abhängig von der Bildung von Homodimeren oder Heterodimeren variieren. Solche Strukturen können sich ebenfalls für klinische Anwendungen geeignet erweisen.

Die biologischen Eigenschaften der erfindungsgemäßen Proteine, insbesondere das mitogene und osteo-induktive Potential, können z.B. in Assays gemäß Seyedin et al., PNAS 82 (1985), 2267-2271 oder Sampath und Reddi, PNAS 78 (1981), 7599-7603 bestimmt werden.

Ein weiterer Gegenstand der vorliegenden Erfindung ist ein Verfahren zur Herstellung eines Proteins der TGF-β-Familie, welches dadurch gekennzeichnet ist, daß man eine mit einer erfindungsgemäßen DNA oder einem erfindungsgemäßen Vektor transformierte Wirtszelle kultiviert und das TGF-β-Protein aus der Zelle oder/und dem Kulturüberstand gewinnt. Ein solches Verfahren umfaßt die Kultivierung der transformierten Wirtszelle in einem geeigneten Kulturmedium und die Reinigung des erzeugten TGF-β-artigen Proteins. Auf diese Weise ermöglicht das Verfahren die Herstellung einer ausreichenden Menge des gewünschten Proteins zum Einsatz bei der medizinischen Behandlung oder in Anwendungen unter Verwendung von Zellkulturtechniken, bei denen Wachstumsfaktoren benötigt werden. Die Wirtszelle kann ein Bakterium, wie etwa Bacillus oder E.coli, ein Pilz, wie etwa Hefe, eine Pflanzenzelle, wie etwa Tabak, Kartoffel oder Arabidopsis oder eine tierische Zelle, insbesondere eine Wirbeltierzellinie, wie etwa Mo-, COS- oder CHO-Zellinien oder eine Insektenzellinie sein.

Noch ein weiterer Gegenstand der vorliegenden Erfindung ist die Bereitstellung von pharmazeutischen Zusammensetzungen, die eine pharmazeutisch wirksame Menge eines erfindungsgemäßen TGF-β-artigen Proteins als Wirkstoff enthalten. Gegebenenfalls umfaßt eine solche Zusammensetzung einen pharmazeutisch aktzeptablen Träger-, Hilfs-, Verdünnungs- oder Füllstoff. Eine solche pharmazeutische Zusammensetzung kann bei der Wundheilung und Gewebewiederherstellung sowie bei der Heilung von Knochen-, Knorpel-, Bindegewebs-, Haut-, Schleimhaut-, Epithelial- oder Zahnschädigungen und bei Zahnimplantaten entweder alleine oder in Kombination mit anderen Wirkstoffen, z.B. anderen Proteinen der TGF-β-Familie oder Wachstumsfaktoren, wie etwa EGF (epidermal growth factor) oder PDGF (platelet derived growth factor) verwendet werden. Ferner kann eine solche pharmazeutische Zusammensetzung bei der Krankheitsprävention, wie z.B. zur Prävention von Osteoporose und Arthrose verwendet werden.

Eine andere mögliche klinische Anwendung des erfindungsgemäßen TGF-β-artigen Proteins ist die Verwendung als Suppressor der Immunreaktion zur Vermeidung der Abstoßung von Organtransplantaten oder ein Einsatz im Zusammenhang mit der Angiogenese. Die erfindungsgemäße pharmazeutische Zusammensetzung kann auch prophylaktisch oder in der kosmetischen Chirurgie verwendet werden. Weiterhin ist die Anwendung der Zusammensetzung nicht auf Menschen beschränkt, sondern kann auch Tiere, insbesondere Haustiere umfassen.

Schließlich ist ein weiterer Gegenstand der vorliegenden Erfindung ein Antikörper, der spezifisch an die erfindungsgemäßen Proteine binden kann, oder ein derartiges Antikörperfragment (z.B. Fab oder Fab'). Verfahren zur Herstellung eines solchen spezifischen Antikörpers oder Antikörperfragments gehören zum allgemeinen Fachwissen des Durchschnittsfachmanns. Vorzugsweise ist ein solcher Antikörper ein monoklonaler Antikörper. Solche Antikörper oder Antikörperfragmente könnten sich auch für diagnostische Methoden eignen.

Weiterhin soll die Erfindung durch das folgende Beispiel veranschaulicht werden.

### Beispiel 1

### Isolierung von MP-52

1.1 Gesamt-RNA wurde aus menschlichem Embryonalgewebe (8 bis 9 Wochen alt) nach der Methode von Chirgwin et al., Biochemistry 18 (1979), 5294-5299 isoliert. Poly(A+)-RNA wurde aus der Gesamt-RNA durch Oligo (dT)-Chromatographie gemäß den Vorschriften des Herstellers (Stratagene Poly (A) Quick-Säulen) abgetrennt.
1.2 Für die reverse Transkriptionsreaktion wurden 1 bis 2,5 µg Poly (A+)-RNA für 5 Minuten auf 65°C erhitzt und schnell auf Eis abgekühlt. Das Reaktionsgemisch enthielt 27 U RNA-Guard (Pharmacia), 2,5 µg Oligo (dT)12-18. (Pharmacia), 5 x Puffer (250 mmol/l Tris/HCl pH 8,5, 50 mmol/l MgCl2, 50 mmol/l DTT, 5 mmol/l von jedem dNTP, 600 mmol/l KCl) und 20 U AMV reverse Transkriptase (Boehringer Mannheim) pro µg Poly (A+) RNA. Das Reaktionsgemisch (25 µl) wurde 2 Stunden lang bei 42°C inkubiert.
1.3 Die in Fig. 2 gezeigten Deoxynukleotidprimer OD und OID wurden auf einem automatischen DNA-Synthesizer (Biosearch) hergestellt. Die Reinigung erfolgte durch denaturierende Polyacrylamidgelelektrophorese und Isolierung der Hauptbande aus dem Gel durch Isotachophorese. Die Oligonukleotide wurden durch Vergleich der Nukleinsäuresequenzen von bekannten Mitgliedern der TGF-β-Familie und Auswahl von Regionen mit der höchsten Konservierung entworfen. Ein Vergleich dieser Region ist in Fig. 2 gezeigt. Zur Erleichterung der Klonierung enthielten beide Nukleotide EcoRI-Restriktionsstellen und OD enthielt zusätzlich eine NcoI-Restriktionsstelle an seinem 5'-Terminus.
1.4 Bei der PCR-Reaktion wurde 20 ng Poly (A+) RNA entsprechende cDNA aus Ausgangsmaterial verwendet. Die Reaktion wurde in einem Volumen von 50 µl durchgeführt und enthielt 1 x PCR-Puffer (16,6 mmol/l (NH4)2SO4, 67 mmol/l Tris/HCl pH 8,8, 2 mmol/l MgCl2, 6,7 µmol/l EDTA, 10 mmol/l β-Mercaptoethanol, 170 µg/ml Rinderserumalbumin (Gibco), 200 µmol/l von jedem dNTP (Pharmacia), 30 pmol von jedem Oligonukleotid (OD und OID) und 1,5 U Taq-Polymerase (AmpliTag, Perkin Elmer Cetus). Das Reaktionsgemisch wurde mit Paraffin überschichtet und es wurden 40 PCR-Zyklen durchgeführt. Die Produkte der PCR-Reaktion wurden durch Phenol/Chloroform-Extraktion gereinigt und durch Ethanolpräzipitation konzentriert.
1.5 Das PCR-Reaktionsprodukt wurde mit den Restriktionsenzymen SphI (Pharmacia) und AlwNI (Biolabs) entsprechend den Vorschriften des Herstellers gespalten.
1.6 Die Produkte der Restriktionsspaltung wurden durch Agarosegelelektrophorese fraktioniert. Nach Anfärbung mit Ethidiumbromid wurden nicht gespaltene Amplifizierungsprodukte aus dem Gel herausgeschnitten und durch Phenolextraktion isoliert. Die erhaltene DNA wurde anschließend zweimal durch Phenol/Chloroform-Extraktion gereinigt.
1.7 Nach einer Ethanolpräzipitation wurde ein Viertel oder ein Fünftel der isolierten DNA reamplifiziert, wobei die gleichen Bedingungen wie für die primäre Amplifikation verwendet wurden, außer daß die Anzahl der Zyklen auf 13 verringert wurde. Die Reamplifizierungsprodukte wurden gereinigt, mit den gleichen Enzymen wie oben geschnitten und die ungeschnittenen Produkte wurden, wie oben für die Amplifizierungsprodukte erläutert, aus Agarosegelen isoliert. Der Reamplifizierungsschritt wurde zweimal wiederholt.
1.8 Nach der letzten Isolierung aus dem Gel wurden die Amplifizierungsprodukte durch 4 U EcoRI (Pharmacia) unter den vom Hersteller empfohlenen Bedingungen gespalten. Ein Viertel des Restriktionsgemisches wurde in den mit EcoRI gespaltenen Vektor pBluescriptII SK+ (Stratagene) ligiert. Nach Ligierung wurden 24 Klone durch Sequenzierung weiter analysiert. Die mit AlwNI und SphI gespaltene Probe ergab eine neue Sequenz, die als MP-52 bezeichnet wurde. Die anderen Klone enthielten überwiegend BMP6-Sequenzen und einer enthielt eine BMP7-Sequenz.

Der Klon wurde zum 3'-Ende der cDNA nach der ausführlich von Frohmann (Amplifications, veröffentlicht von Perkin-Elmer Corp., Issue 5 (1990), pp 11-15) beschriebenen Methode vervollständigt. Die gleiche embryonale mRNA, die zur Isolierung des ersten Fragments von MP-52 verwendet worden war, wurde, wie oben beschrieben, revers transkribiert. Die Amplifizierung erfolgte unter Verwendung des Adapterprimers (AGAATTCGCATGCCATGGTCGACG) und eines inneren Primers (CTTGAGTACGAGGCTTTCCACTG) der MP-52-Sequenz. Die Amplifizierungsprodukte wurden unter Verwendung eines überlappenden Adapterprimers (ATTCGCATGCCATGGTCGACGAAG) und eines überlappenden internen Primers (GGAGCCCACGAATCATGCAGTCA) der MP-52-Sequenz reamplifiziert. Die Reamplifizierungsprodukte wurden nach Restriktionsspaltung mit NcoI in einen auf gleiche Weise gespaltenen Vektor (pUC 19 (Pharmacia Nr. 27-4951--01) mit einer geänderten multiplen Klonierungsstelle, die eine singuläre NcoI-Restriktionsstelle enthält) kloniert und sequenziert. Die Klone wurden durch ihre Sequenzüberlappung am 3'-Ende der bekannten MP-52-Sequenz charakterisiert. Einer davon wurde als Sonde zum Screening einer humanen genomischen Genbank (Stratagene Nr. 946203) nach einer ausführlich bei Ausubel et al. (Current Protocols in Molecular Biology, veröffentlicht von Greene Publishing Associates und Wiley-Interscience (1989)) beschriebenen Methode verwendet. Aus 8 x 10⁵ λ Phagen wurde ein Phage (λ2.7.4) isoliert, der eine Insertion von etwa 20 kb enthielt, und bei DSM unter der Hinterlegungsnummer 7387 hinterlegt. Dieser Klon enthält neben der aus mRNA durch die beschriebenen Amplifizierungsmethoden isolierte Sequenz weitere Sequenzinformationen am 5'-Ende.

Zur Sequenzanalyse wurde ein HindIII-Fragment von etwa 7,5 kb in einen auf gleiche Weise geschnittenen Vektor (Bluescript SK, Stratagene Nr. 212206) subkloniert. Dieses als SKL 52 (H3) MP12 bezeichnete Plasmid wurde ebenfalls bei DSM unter der Hinterlegungsnummer 7353 hinterlegt. Die in SEQ ID NO. 1 gezeigte Sequenzinformation stammt von dem Phagen λ 2.7.4.. Das ATG an Position 640 ist das erste ATG innerhalb des Leserahmens (bei Position 403 tritt ein Stoppkodon auf). Aufgrund der Sequenzdaten ist zu vermuten, daß es sich hierbei um das Startkodon für die Translation handelt.

Die genomische DNA enthält ein Intron von etwa 2 kb zwischen den Basenpaaren 1270 und 1271 von SEQ ID NO. 1. Die Sequenz des Introns ist nicht gezeigt. Die Richtigkeit der Splicestelle wurde durch Sequenzierung eines Amplifizierungsprodukts bestätigt, das aus einer diese Region enthaltenden cDNA stammt. Diese Sequenzinformationen wurden mit Hilfe einer leicht modifizierten Methode erhalten, die ausführlich bei Frohman (Amplifications, veröffentlicht von Perkin-Elmer Corporation, Issue 5 (1990), pp 11-15) beschrieben ist. Es wurde die gleiche embryonale RNA, die auch zur Isolierung des 3'-Endes von MP-52 verwendet wurde, unter Einsatz eines internen, in 5'-Richtung orientierten Primers der MP-52-Sequenz (ACAGCAGGTGGGTGGTGTGGACT) revers transkribiert. Ein PolyA-Schwanz wurde an das 5'-Ende des ersten cDNA-Strangs unter Verwendung terminaler Transferase angefügt. Es wurde eine 2-Schritt-Amplifizierung durchgeführt, zuerst durch Verwendung eines aus Oligo dT und einer Adaptersequenz bestehenden Primers (AGAATTCGCATGCCATGGTCGACGAAGC(T16)) und zweitens eines Adapterprimers (AGAATTCGCATGCCATGGTCGACG) und eines internen Primers (CCAGCAGCCCATCCTTCTCC) der MP-52-Sequenz durchgeführt. Die Amplifizierungsprodukte wurden unter Verwendung des gleichen Adapterprimers und eines überlappenden internen Primers (TCCAGGGCACTAATGTCAAACACG) der MP-52-Sequenz reamplifiziert. Anschließend wurden die Reamplifizierungsprodukte unter Verwendung eines überlappenden Adapterprimers (ATTCGCATGCCATGGTCGACGAAG) und eines überlappenden internen Primers (ACTAATGTCAAACACGTACCTCTG) der MP-52-Sequenz reamplifiziert. Die Reamplifizierungsendprodukte wurden mit glatten Enden in einen Vektor (Bluescript SK, Stratagene Nr. 212206) kloniert, der mit EcoRV gespalten war. Die Klone wurden durch ihre Sequenzüberlappung mit der DNA von λ 2.7.4. charakterisiert.

Weiterhin wurde eine cDNA-Bank, hergestellt aus RNA von humanen Fibroblasten und kloniert in λ gt10, gescreent. Dabei wurden 2 x 106 Phagen getestet, wobei als radioaktive Sonde ein ca. 1 kb großes Fragment der genomischen MP-52-DNA (2. Exon bis zur HindIII-Restriktionsstelle im 3'-untranslatierten Bereich) diente. Es wurden 17 Mischplaques gepickt, die mit PCR unter Verwendung von Primern aus dem 5'- und 3'-Bereich der MP-52-Sequenz überprüft wurden. Daraufhin wurden 8 Phagenplaques ausgewählt und vereinzelt. Die cDNA wurde über eine EcoRI-Partialspaltung aus dem Phagen isoliert und in den ebenfalls mit EcoRI gespaltenen Bluescriptvektor kloniert.

Eine Sequenzierung eines der resultierenden Plasmide SK52L15.1MP25, zeigte, daß der längste Phage (15.1) bei Nukleotid Nr. 321 von SEQ ID NO. 1 beginnt. Weiterhin wurde durch das Sequenzieren die Splicestelle (Nukleotid 1270) bestätigt.

Das Plasmid SKL 52 (H3) MP12 wurde unter der Hinterlegungsnummer 7353 bei DSM (Deutsche Sammlung von Mikroorganismen und Zellkulturen, Mascheroder Weg 1b, 38124 Braunschweig) am 10. Dezember 1992 hinterlegt.

Der Phage λ 2.7.4 wurde unter der Hinterlegungsnummer 7387 bei DSM am 13. Januar 1993 hinterlegt.

Der Plasmid SK52L15.1MP25 wurde unter der Hinterlegungsnummer 8421 bei DSM am 16. Juli 1993 hinterlegt.

### Beispiel 2

### Expression von MP52

Für die Expression von MP52 wurden verschiedene Systeme getestet. Die Verwendung von Vaccinia Viren als Expressionssystem ist ausführlich und für den Fachmann nacharbeitbar in den Current Protocols in Molecular Biology (Ausubel et al., Greene Publishing Associates and Wiley-Interscience, Wiley & Sons) im folgenden abgekürzt mit CP unter Chapter 16 Unit 16.15-16.18 beschrieben. Das System beruht darauf, daß Fremd-DNA unter Verwendung bestimmter Vektoren durch homologe Rekombination in das Vaccinia Virus Genom integriert werden kann. Zu diesem Zweck enthält der verwendete Vektor das TK (Thymidinkinase)-Gen aus dem Vaccinia Genom. Um eine Selektion auf rekombinante Viren zu ermöglichen, enthält der Vektor weiterhin das E.coli-Xanthin-Guanin-Phosphoribosyl-Transferase-Gen (gpt) (Falkner et al., J. Virol. 62 (1988), 1849-1854). In diesen Vektor wurde die cDNA mit dem gesamten codierenden Bereich für MP52 kloniert. Die cDNA kommt aus dem Plasmid SK52L15.1MP25 (DSM, Hinterlegungsnummer 8421), welche zur Entfernung eines großen Teils des 5'-nicht translatierten Bereichs aber zunächst deletiert und zwischenkloniert wurde. Dazu wurde das Plasmid SK52L15.1MP25 mit SalI linearisiert und stufenweise das 5'-Ende mit dem ExoIII/Mung Bean Kit (Stratagene #200330) nach Herstellerangaben deletiert. Nach Restriktion mit BamHI wurden die unterschiedlich weit deletierten MP52 cDNAs über ein Agarosegel von dem Restvektor getrennt, isoliert und nach Standardmethoden (Sambrook et al., Molecular Cloning, second edition, Cold Spring Harbor Laboratory Press 1989) in einem mit EcoRV und BamHI restringierten pBluescriptII SK- Vektor (Stratagene #212206) zwischenkloniert (pSK52s). Sämtliche Restriktionen erfolgten nach Herstellerangaben. Ansequenzierung mit Sequenase (USB/Amersham #70770) ergab unter anderem einen Klon, der mit Nukleotid 576 in SEQ ID NO.1 (64 Basenpaare vom Startcodon entfernt) beginnt. Aus diesem wurde über SalI und SacI Restriktion das cDNA-Insert isoliert und in den ebenso gespaltenen Vektor für die Rekombination in Vaccinia kloniert. Das resultierende Plasmid (pBP1MP52s) wurde bei der DSM (Hinterlegungsnummer 9217) am 24. Mai 1994 hinterlegt und für die Herstellung von rekombinanten Vaccinia Viren eingesetzt. Dazu wurden zu 80 % konfluente 143B Zellen (HuTk-, ATCC CRL 8303) in 35 mm Kulturschalen mit Vaccinia Wildtyp Virus in 2 ml PBS für 30 Minuten bei Raumtemperatur unter gelegentlichem Schütteln infiziert (1 Virus auf 10 Zellen). Nach Absaugen des Überstandes und Zugabe von 2 ml Kulturmedium (MEM, Gibco BRL #041-01095) wurde für 2 Stunden bei 37°C inkubiert. Das Medium wurde anschließend entfernt und die Transformation dieser Zellen mit 100 ng pBP1MP52s, 2 µg Träger DNA (Kalbsthymus, Boehringer Mannheim #104175) und 10 µl Lipofektin (Gibco BRL #18292-011) in 1 ml MEM für 15 h bei 37°C erreicht. Nach Zugabe von 1 ml MEM mit 20 % FCS (Gibco BRL #011-06290) wurde für weitere 24 Stunden bei 37°C inkubiert und die lysierten Zellen anschließend eingefroren.

Die gpt Selektion auf die Xanthin-Guanin-Phosphoribosyl-Transferase und Isolation und Amplifizierung einzelner rekombinanter Viren erfolgte im wesentlichen wie in Unit 16.17 der CP beschrieben, mit dem Unterschied, daß RK13-Zellen (ATCC CCL 37) verwendet wurden.

Die Integration der MP52 cDNA in das Virus-Genom wurde durch Dot blot und Southern blot Analyse (CP Unit 16.18) bestätigt. Ein rekombinantes Virus wurde für Expressionsanalysen in der Zellinie 143B (HuTk-, ATCC CRL 8303, human) eingesetzt. Die konfluenten Zellen wurden mit der der Zellzahl entsprechenden Anzahl an Viren für 45 Minuten bei 37°C infiziert und anschließend das entsprechende Kulturmedium (MEM, Gibco BRL #041-01095) mit 10 % FCS und Penicillin/Streptomycin (1:500, Gibco BRL #043-05140H) zugefügt. Nach 6 Stunden bei 37°C wurde das Medium entfernt, die Zellen zweimal mit z.B. HBSS (Gibco BRL #042-04180M) gewaschen und Produktionsmedium (z.B. MEM) ohne FCS zugesetzt. Nach 20 bis 22 Stunden Produktion wurde der Zellüberstand gesammelt. Die Analyse der Expression erfolgte durch Western blots nach Standardmethoden (CP Unit 10.8). Dafür wurden die Proteine aus 100 bis 500 µl Zellkulturüberstand durch Zugabe des äquivalenten Volumens an Aceton und Inkubation von mindestens einer Stunde auf Eis präzipitiert und abzentrifugiert. Nach Resuspension des Pellets in Auftragspuffer (7 M Harnstoff, 1 % SDS, 7 mM Natriumdihydrogenphosphat, 0,01 % Bromphenolblau und gegebenenfalls 1 % β-Mercaptoethanol) erfolgte die Auftrennung in 15 %igen Polyacrylamidgelen. Als Markerproteine wurde ein vorgefärbter Protein-Molekulargewichtsstandard (Gibco BRL #26041-020) eingesetzt. Der Transfer auf PVDF-Membran (Immobilon #IPVH00010) und das Abblocken der Membran erfolgten nach Standardmethoden.

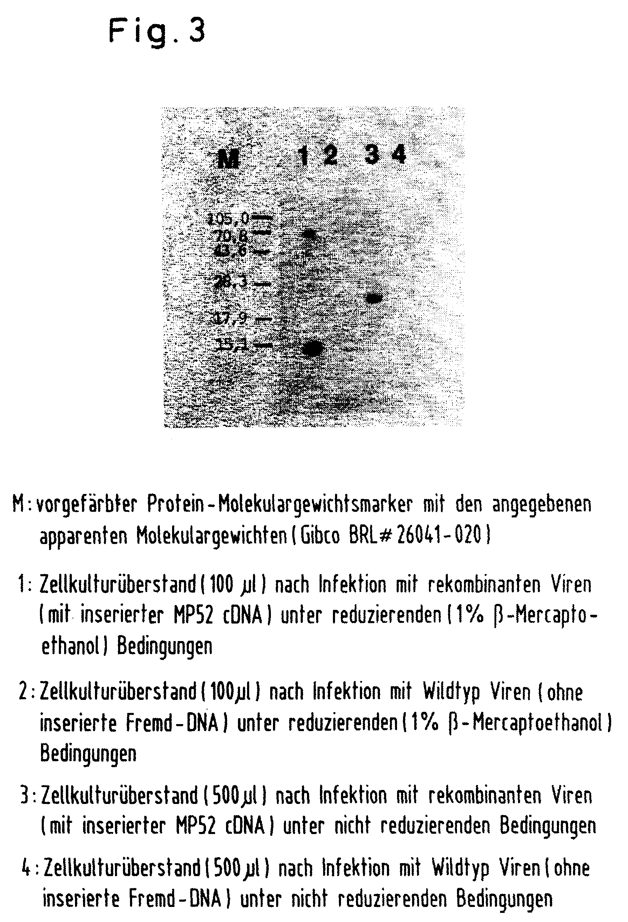

Zur Detektion von MP52 auf der Membran waren polyklonale Antikörper gegen MP52 sowohl in Hühnern als auch in Kaninchen erzeugt worden. Dazu wurde der reife Anteil von MP52 mit 6 Histidinen am N-Terminus in E.coli exprimiert und gereinigt, wie z.B. beschrieben in Hochuli et al. (BIO/Technology, Vol. 6, 1321-1325 (1988)). Mit beiden Antikörpern ist es möglich, spezifisch Expression von MP52 nachzuweisen, wobei dimeres MP52 weniger effizient erkannt wird als monomeres. Für den Western blot in Figur 3 wurden Hühner-Antikörper verwendet, die über PEG-Präzipitation (Thalley et al., BIO/Technology Vol. 8, 934-938 (1990)) und über Membran-gebundenes Antigen (reifes MP52 mit 6 Histidinen) (18.17 in Sambrook et al., Molecular Cloning, second edition, Cold Spring Harbor Laboratory Press 1989) spezifisch gereinigt waren. Als zweiter Antikörper wurde Anti-Chicken IgG mit gekoppelter alkalischer Phosphatase (Sigma A9171) eingesetzt. Die Detektion erfolgte mit dem Tropix Western-Light Protein Detection Kit (Serva #WL10RC) nach Herstellerangaben.

Der Western blot in Figur 3 zeigt, daß nur bei den rekombinanten Viren, nicht aber bei den Wildtyp Viren (ohne integrierte Fremd-DNA) MP52 spezifische Banden auftreten. Die Expression von MP52 führt zu einem sekretierten Protein mit einem im Gel unter nicht reduzierenden Bedingungen erscheinenden Molekulargewicht von ungefähr 25 kDa. Unter reduzierenden Bedingungen läuft das Protein bei 14 bis 15 kDa im Gel. Diese Ergebnisse zeigen, daß MP52 als dimeres reifes Protein exprimiert wird. Bei den im Western blot auftretenden schwachen Banden im Bereich oberhalb von 60 kDa handelt es sich wahrscheinlich um Reste von ungeschnittenen Vorläuferproteinen. Das Laufverhalten bestätigt zudem die aus SEQ ID NO.2 abzuleitenden theoretischen Molekulargewichte, wonach reifes, monomeres MP52 eine Größe von 13.6 kDa besitzt.

Die Expression von MP52 und Spaltung des Vorläuferproteins zum reifen MP52 ist nachweislich in verschiedenen Zellinien möglich. Getestet wurden C127 (ATCC CRL 1616, Maus), BHK21 (ATCC CCL 10, Hamster), MRC-5 (ATCC CCL 171, Mensch) und 3T6-Swiss albino (ATCC CCL 96, Maus) Zellen.

Expression und Spaltung zum reifen MP52 wurde auch in einem weiteren eukaryontischen Expressionssystem gezeigt. Dafür wurde die cDNA von MP52 (beginnend mit Nukleotid 576) in das Expressionsplasmid pSG5 (Stratagene #216201) kloniert. Das Plasmid pSK52s wurde mit ClaI und XbaI restringiert und durch T4-Polymerasebehandlung die überhängenden Enden des MP52-Inserts stumpf gemacht. Die Klonierung in den mit EcoRI restringierten und durch T4-Polymerasebehandlung ebenfalls stumpfendigen Vektor pSG5 erfolgte nach Standardmethoden. Alle enzymatischen Reaktionen erfolgten nach Herstellerangaben. Die korrekte Orientierung des MP52-Inserts wurde durch Restriktionsanalyse und Ansequenzierung mit dem T7-Primer (Stratagene #300302) abgesichert. Das resultierende Plasmid pSG52s (am 17.05.94 bei der DSM mit der Hinterlegungsnummer DSM 9204 hinterlegt) kann mit einem Vektor, der für einen selektierbaren Marker kodiert, wie z.B. das Gen für G418-Resistenz, kotransformiert werden, um stabile Zellinien zu erhalten. Zu diesem Zweck wurde pSG52s mit dem Plasmid p3616 (am 17.05.94 bei der DSM mit der Hinterlegungsnummer DSM 9203 hinterlegt) in L929 Zellen (ATCC CCL1, Maus) mit Lipofektin (Gibco BRL #18292-011) nach Herstellerangaben kotransformiert. Die Selektion mit G418 erfolgte nach dem Fachmann bekannten Methoden (CP, unit 9.5) und führte zu einer Zelllinie, die im Western blot nachweisbar reifes MP52 produziert.

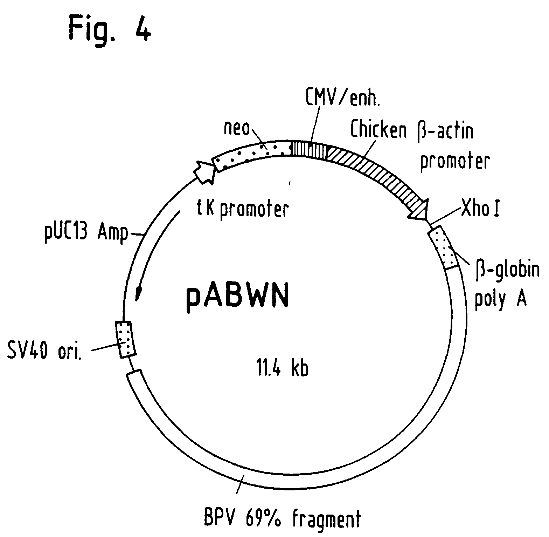

Ein weiterer Expressionsvektor für MP52 wurde unter Verwendung des Plasmides pABWN (Niwa et al., Gene 108 (1991), 193-200 und Figur 4), das von Dr. Miyazaki zur Verfügung gestellt wurde, hergestellt.

Dazu wurde das Hind III Fragment aus dem Plasmid pSK52s, das mit dem Nukleotid 576 in SEQ ID NO. 1 beginnt, isoliert und die überhängenden Enden durch Behandlung mit Klenow Fragment stumpfendig gemacht. Durch Ligation des Adapters wurde an beiden Fragmentenden eine Not I Restriktionsschnittstelle eingeführt.
- Adapter:: AGCGGCCGCT
TCGCCGGCGA

Der Vektor pABWN wurde mit Xho I restringiert, ebenfalls mit dem Klenow Fragment behandelt und mit der intestinalen Alkalischen Phosphatase vom Kalb (Boehringer Mannheim) dephosphoryliert. Derselbe phosphorylierte Adapter wurde anligiert, so daß nun eine Insertion des MP52-Fragmentes nach Restriktion mit Not I in die generierte Not I Schnittstelle des Vektors möglich war. Der resultierende Expressionsvektor wird nachfolgend als Hind III-MP52/pABWN bezeichnet. Alle durchgeführten Reaktionen für die Klonierung erfolgten nach Standardmethoden (z.B. CP unit 3.16).
Die Struktur des Hind III-MP52/pABWN Expressionsvektors wurde durch Sequenzierung und Restriktionskartierung bestätigt. Hind III-MP52/pABWN enthält die MP52-Sequenz beginnend mit Nukleotid 576 und endend mit Nukleotid 227 8 in SEQ ID NO. 1.

HindIII-MP52/pABWN wurde in L-Zellen (Maus-Fibroblasten) transfiziert, und es wurden daraus stabile Transformanten etabliert. Dazu wurden jeweils 4 µg der Plasmide (Hind III-MP52/pABWN oder pABWN) in 5 x 10⁵ L-Zellen auf einer 6 cm Kulturschale unter Verwendung von 20 µl LipofectAMINE Reagenz (Gibco BRL #18324-012) transfiziert. Dazu wurde Lösung A (4 µg der jeweiligen Plasmid DNA in 200 µl OPTI-MEM I (Gibco BRL # 31985)) vorsichtig gemischt mit Lösung B (20 µl LipofectAMINE Reagenz in 200 µl OPTI-MEM I) und bei Raumtemperatur für 45 Minuten zur Bildung des DNA-Liposomen Komplexes inkubiert. Während dessen wurden die Zellen einmal mit 2 ml OPTI-MEM I gewaschen. Für jede Transfektion wurden 1,6 ml OPTI-MEM I zu dem Gefäß mit dem DNA-Liposomen Komplex gegeben. Die Lösung wurde vorsichtig gemischt und damit die gewaschenen Zellen überschichtet. Die Zellen wurden mit dem verdünnten Komplex für 5 Stunden bei 37°C im CO₂ Inkubator inkubiert. Nach der Inkubation wurden 2 ml DMEM (Gibco BRL, Dulbecco's Modifiziertes Eagle Medium) / 20% FCS zugegeben. 24 Stunden nach der Transfektion wurde das Medium mit frischem DMEM/10% FCS ersetzt. 48 Stunden nach Beginn der Transfektion wurden die Zellen in eine 10 cm Kulturschale überführt. 72

Stunden nach Beginn der Transfektion wurde die G418 Selektion mit einer Konzentration von 800 µg/ml begonnen. Die stabilen Klone erschienen nach 1 bis 2 Wochen.

5 ml konditioniertes DMEM mit oder ohne FCS wurde von konfluenten Transformanten erhalten, die 3 Tage in einer 10 cm Kulturschale gewachsen waren. Die 2 verschiedenen Zellkulturüberstände (HindIII-MP52/pABWN und pABWN) transfizierter Zellen sowie Zellysate wurden im Western blot untersucht. Dabei wurde reifes MP52 in konditioniertem Medium sowie in Zellysaten von HindIII-MP52/pABWN transfizierten Zellen gefunden. Die Klone wurden weiterkloniert und MP52 produzierende Zellen jeweils nach Western blot Analyse ausgewählt. Abschätzungen aus Western blot Analysen ergaben MP52 Produktion von bis zu 1 mg/l.

### Beispiel 3:

### Biologische Aktivität von MP52

Um die biologische Aktivität von MP52 nachzuweisen und die Nützlichkeit dieser Erfindung für medizinische Anwendungen zur Vermeidung und/oder Behandlung von Knochenkrankheiten zu belegen, wurden mehrere Experimente *in vitro* und *in vivo* durchgeführt.

### 1. In vitro assays

### 1.1

Da eine Steigerung der Glykosaminoglykan (GAG) Synthese in Chondrozyten nach TGF-β Stimulation beschrieben ist (Hiraki et al., Biochimica et Biophysica Acta 969 (1988), 91-99), wurde untersucht, ob MP52 ebenfalls diesen Einfluß ausübt. Unter Verwendung der Zellkulturüberstände (DMEM mit 10% FCS) von MP52 produzierenden L-Zelltransformanten (transfiziert mit Hind III-MP52/pABWN), wurde die chondrogene Aktivität von MP52 in Primärkulturen aus fötalen Rattenextremitäten getestet.

Dazu wurden die vier Extremitäten von 16-Tage alten Rattenföten verwendet. Nach Trypsinierung wurden die gewonnenen Zellen in F-12 Medium (Nutrient Mixture Ham's F-12, Gibco BRL #21700) mit 10% FCS auf Kollagen-Typ I beschichteten 24-Well Platten mit 3x10⁵ Zellen ausplattiert und ca. 2 Tage bis zur Konfluenz kultiviert. Zu 500 µl Kulturmedium (F-12 Medium mit 10% FCS) wurden jeweils 56 µl konditioniertes Medium (KM) von HindIII-MP52/pABWN-L-Zelltransfektanten, von pABWN-L-Zelltransfektanten oder nur Medium (DMEM mit 10% FCS) gegeben. Über einen Zeitraum von 0, 3, 6 und 9 Tagen wurde F-12 Medium mit 10% FCS sowie den entsprechenden Zusätzen verwendet. Alle drei Tage erfolgte ein Wechsel des Mediums mit den entsprechenden Zusätzen. Danach wurde die Kultur für weitere 2 Tage in F-12 Medium ohne FCS in Anwesenheit der entsprechenden Zusätze (konditionierte Medien bzw. Kontrollmedium) kultiviert und dann ³⁵S-Sulfat für 6 Stunden zugesetzt. In Polysaccharide inkorporiertes ³⁵S wurde nach Pronase E Verdau und Präzipitation wie in Hiraki et al. (Biochimica et Biophysica Acta 969 (1988), 91-99) beschrieben, gemessen.

**Tabelle 1**

| | Radioaktivität (cpm/well) | | |
|---|---|---|---|
| Anzahl der Inkubationstage | DMEM (10%FCS) von Kontroll-L-Zellen | KM von pABWN-L-Zelltransfektanten | KM von HindIII-MP52/pABWN-L-Zelltransfektanten |
| 2 | 3720±114 | 3865±120 | 4879±422 |
| 5 | 4188±135 | 4154±29 | 8223±275* |
| 8 | 3546±160 | 3310±115 | 9890±1260* |
| 11 | 3679±218 | 3633±167 | 7520±160* |
| Werte beziehen sich auf ± S.E.M. für 3 oder 4 Kulturansätze | | | |

| | | | |
|---|---|---|---|
| *: p<0,01 vs DMEM und KM von pABWN-L-Zelltransfektanten (Scheffe's multiple t-test) | | | |

Wie in Tabelle 1 gezeigt, stimulieren die Zellkulturüberstände der MP52 produzierenden Transfektanten signifikant die GAG Synthese im Vergleich zu reinem Kulturmedium (DMEM mit 10% FCS) oder dem Zellkulturüberstand von pABWN-transfizierten L-Zellen. Dies zeigt, daß MP52 die Chondrozytendifferenzierung stimulieren kann.

### 1.2

Ein beschriebener Effekt für einige Mitglieder der BMP-Familie ist die Steigerung der alkalische Phosphatase (ALP)-Aktivität in Osteoblasten. Die klonale Ratten-Zellinie ROB-C26 (C-26) zählt zu den Osteoblasten eines relativ frühen Reifestadiums (Yamaguchi et al., Calcif. Tissue Int. 49 (1991), 221-225). Für osteoinduktive Proteine wie z.B. das BMP-2 ist die Fähigkeit zur Steigerung der ALP-Aktivität bei Yamaguchi et al. (J. Cell Biol. 113 (1991), 681-687) beschrieben.

Der Einfluß von MP52 auf C26-Zellen wurde wie folgt untersucht: Die C26-Zellen wurden mit 3 x 10⁴ Zellen pro Well in eine 24-Well Platte ausgesät und in α-MEM (Gibco BRL) / 10% FCS bis zur Konfluenz kultiviert. Pro Well wurden 56 µl des Zellkulturüberstandes von MP52 produzierenden L-Zelltransfektanten (Hind III-MP52/pABWN) bzw. Zellkulturüberstand von pABWN-L-Zelltransfektanten oder nur Zellkulturüberstand (DMEM mit 10% FCS) von L-Zellen zu 500 µl des C-26 Zellkulturmediums gegeben. Ein Mediumwechsel mit den entsprechenden Zusätzen erfolgte alle drei Tage. Die ALP-Aktivität in den Zellextrakten wurde nach 0, 3, 6, 9 und 12 Tagen mit Hilfe von Standardtechniken basierend auf p-Nitrophenyl-Phosphat als Substrat, wie z.B. beschrieben bei Takuwa et al. (Am. J. Physiol. 257 (1989), E797-E803), bestimmt.

**Tabelle 2**

| | ALP-Aktivität (nmol/min) pro Well | | |
|---|---|---|---|
| Anzahl der Inkubationstage | DMEM (10%FCS) von Kontroll-L-Zellen | KM von pABWN-L-Zelltransfektanten | KM von HindIII-MP52/pABWN-L-Zelltransfektanten |
| 0 | 41,8±2,8 | 41,8±2,8 | 41,8±2,8 |
| 3 | 136,3±3,7 | 125,8±2,3 | 181,3±14,2* |
| 6 | 129,0±7,8 | 119,3±6,4 | 258,0±8,3* |
| 9 | 118,4±3,7 | 110,1±2,8 | 258,4±10,6* |
| 12 | 121,2±3,2 | 125,3±6,0 | 237,8±11,0* |
| Werte beziehen sich auf ± S.D. für 4 Kulturansätze | | | |

| | | | |
|---|---|---|---|
| *:p<0,01 vs DMEM und KM von pABWN-L-Zelltransfektanten (Scheffe's multiple t-test) | | | |

Wie in Tabelle 2 gezeigt wird, steigt die ALP-Aktivität durch die MP52 Zugabe signifikant im Vergleich zu reinem DMEM/10%FCS Medium und Medium von pABWN-infizierten L-Zellen an. Dieses Ergebnis zeigt, daß MP52 nicht nur die Chondrozyten Differenzierung, sondern auch Osteoblasten Differenzierung und Reifung bewirken kann.
Eine weitere Osteoblastenzellinie (MC3T3-E1, Maus), die wie bei Takuwa et al. (Biochem. Biophys. Res. Com. 174 (1991), 96-101 ) beschrieben durch BMP-2 Behandlung einen Anstieg der ALP-Aktivität zeigt, gibt nach Inkubation mit konditioniertem Medium von MP52 produzierenden L-Zelltransfektanten (Hind III-MP52/pABWN) oder Medium nach MP52 Produktion durch Infektion mit rekombinanten Vaccinia Viren keine Veränderung der ALP-Aktivität. Dies weist darauf hin, daß MP52 z.T. eine von BMP-2 abweichende Zellspezifität besitzt. Unterschiedliche Funktionen bedingt durch verschiedene Zielorte für die einzelnen TGF-β Familienmitglieder können von großer medizinischer Relevanz sein.

### 2. In vivo Experimente

### 2.1

Die aussagekräftigste Möglichkeit Knochenentwicklung zu untersuchen, basiert auf der ektopischen Knochenbildung *in vivo*. Diese kann z.B. durch Implantation von entmineralisierter Knochenmatrix induziert werden (Urist, Science 150 (1965), 893-899). Durch Kombination von inaktiver Matrix mit knocheninduzierenden Proteinen kann der gleiche Prozess induziert werden, wie es z.B. beschrieben ist bei Sampath et al. (PNAS*78 (1981), 7599-7603). Dieser Knochenbildungsprozeß gleicht dem der embryonalen enchondralen Knochenbildung und der adulten Knochenheilung. Somit bietet diese Methode die Möglichkeit, Proteine auf ihre Fähigkeit zur Knocheninduktion *in vivo* zu untersuchen.

Für ein solches Experiment wurde MP52 Protein, welches durch Expression im Vaccinia System (siehe Beispiel 2) gewonnen wurde, teilgereinigt und implantiert.
Dazu wurden 143B Zellen (HuTk-, ATCC CRL 8303) in Kulturschalen und Rollerflaschen bis zur Konfluenz angezogen und, wie in Beispiel 2 für Expressionsanalysen beschrieben, mit rekombinanten Viren infiziert, gewaschen und für ungefähr 20 Stunden MP52 in MEM (Gibco BRL, ca. 1 ml pro 10⁶ Zellen) akkumulieren gelassen. Als Kontrolle erfolgte der gleiche Ansatz durch Infektion mit Wildtyp Viren. Zellkulturüberstand (konditioniertes Medium) von jedem Ansatz wurde gesammelt und zentrifugiert (40000 x g für 30 Minuten bei 4°C). Zur Entfernung der Viren wurden die Überstände über anorganische Filter (0.1 µm Porengröße, Whatman, Anotop 25) filtriert. Im Verlauf der Charakterisierung von MP52 konnte gezeigt werden, daß dieses Protein an Heparin Sepharose bindet. Dieses Verhalten wurde für eine Teilreinigung ausgenutzt. Dazu wurde das filtrierte und zentrifugierte, konditionierte Medium auf eine Endkonzentration von 50 mM Tris pH 7.0, 100 mM NaCl und 6 M Harnstoff gebracht und auf eine Heparin Säule (HiTrap™, Pharmacia #17-0407-01), die äquilibriert war in Puffer A (50 mM Tris pH 7.0, 100 mM NaCl und 6 M Harnstoff), geladen. Die beladene Säule wurde mit Puffer A gewaschen und mit einem linearen Gradienten nach 100 % Puffer B (50 mM Tris pH 7.0, 600 mM NaCl und 6 M Harnstoff) bei einer Durchflußrate von 0.5 ml/min innerhalb von 50 min eluiert (2,5 ml pro Fraktion). Die Verwendung von Harnstoff ist nicht zwingend. Über Western blot Analyse (siehe Beispiel 2) konnte überprüft werden, daß MP52 reproduzierbar hauptsächlich in 2 Fraktionen bei ungefähr 250 bis 400 mM NaCl eluiert. Aliquots dieser Fraktionen wurden ebenfalls in nach Herstellerangaben mit Silber gefärbten 15%igen Polyacrylamidgelen (Silver Stain-II, Daiichi #SE140000) überprüft und die Fraktionen gepoolt. Die vergleichbaren Fraktionen nach Reinigung von konditioniertem Medium nach Infektion mit Wildtyp-Viren wurden nach Analyse in mit Silber gefärbten Gelen ebenfalls gepoolt.
Aus weiteren Untersuchungen zu MP52 ergab sich, daß MP52 auch an Hydroxyapatit bindet. Deshalb ist es prinzipiell möglich, eine zusätzliche Reinigung durch eine Hydroxyapatitsäule zu erreichen, bzw. eine Heparinsäule durch eine Hydroxyapatitsäule (z.B.: BIO-RAD, Econo-pac HTP) zu ersetzen. Denkbar sind für weitere Aufreinigungen auch andere dem Fachmann bekannte Methoden wie z.B. Gelsiebsäulen, Ionenaustauschersäulen, Affinitätssäulen, Metallchelatsäulen oder Säulen basierend auf hydrophobe Wechselwirkungen.
Das über Heparin Sepharose Chromatographie vorgereinigte MP52 Protein bzw. die entsprechend noch kontaminierenden Proteine, die sich auch in den Wildtyp infizierten Zellkulturüberständen befinden, wurden weiter mit Hilfe einer Reversed Phase HPLC aufgereinigt. Dazu wurde eine C8-Säule (Aquapore RP300, Applied Biosystems, Partikelgröße: 7µm, Porengröße: 300Å) equilibriert mit 10% Puffer B (Puffer A: 0,1% Trifluoressigsäure; Puffer B: 90% Acetonitril, 0,1% Trifluoressigsäure). Nach Beladung der Säule mit den gepoolten, MP52 enthaltenden Fraktionen der Heparinsäule wurde ausgiebig mit 10% Puffer B gewaschen. Das gebundene Protein wurde mit folgendem Gradienten eluiert: 10 bis 50% Puffer B über 20 Minuten und 50 bis 100% Puffer B über 50 Minuten. Fraktionen zu 500 µl wurden gesammelt und sowohl im Western blot als auch in mit Silber gefärbten Gelen analysiert. Das MP52 Protein eluiert unter den gewählten Bedingungen ungefähr im Bereich von 55 bis 65 % Acetonitril. Die Fraktionen mit MP52 wurden gepoolt. Das gleiche erfolgte mit den korrespondierenden Fraktionen aus der Kontrollreinigung von Zellkulturüberstand der mit Wildtyp-Viren infizierten Zellen.
* Proc.Natl.Acad.Sci. USA

Auch teilgereinigtes MP52-Protein zeigte in einer nach Western blot Analyse abgeschätzten Konzentration von 50 ng/ml eine deutliche Steigerung der ALP-Aktivität auf ROB-C26-Zellen nach drei Inkubationstagen.

Teilgereinigtes MP52-Protein bzw. Kontrollprotein aus den entsprechend teilgereinigten Zellkulturüberständen nach Wildtyp-Viren-Infektion wurden mit Matrix rekonstituiert und in Ratten implantiert, um die Fähigkeit zur Knorpel- und Knochenbildung unter Beweis zu stellen.

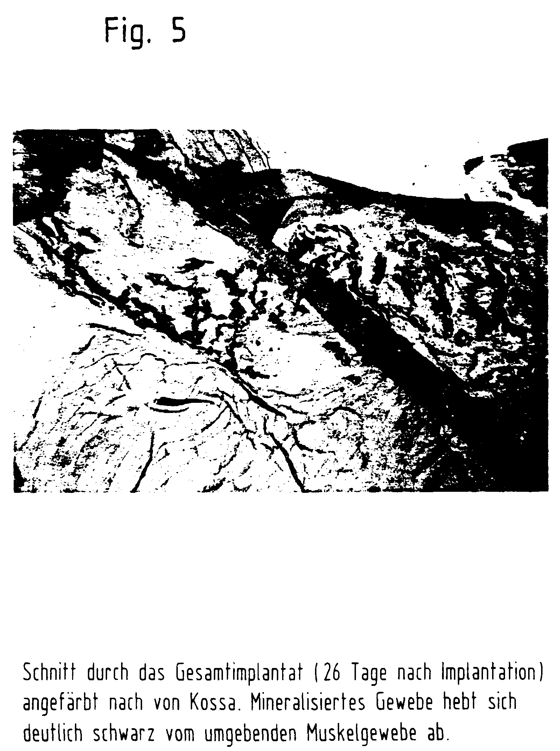

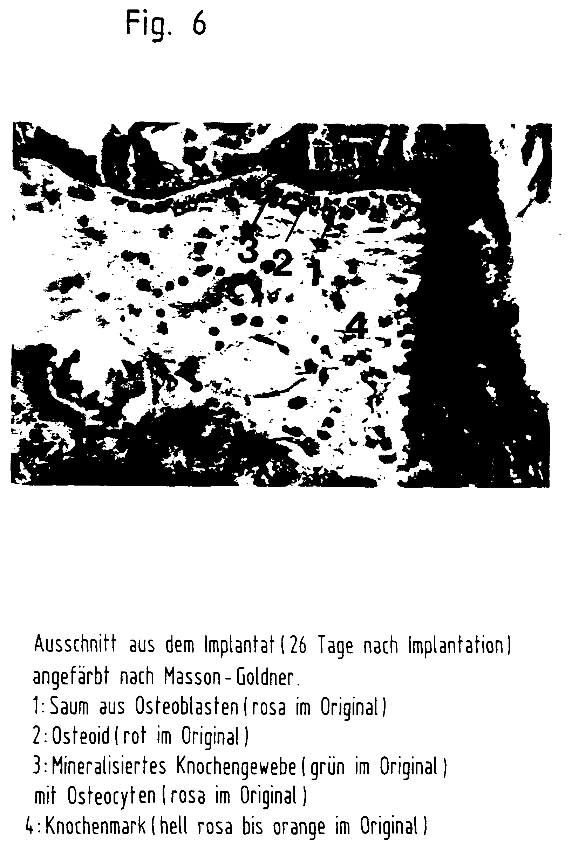

Prinzipiell sollten verschiedene dem Fachmann bekannte Matrixmaterialien verwendbar sein, d.h. natürliche (auch modifizierte) und synthetisch hergestellte Matrices, bevorzugt sind aber biokompatible, *in vivo* biologisch abbaubare poröse Materialien. In diesen Experimenten wurde Knochenmatrix von Ratten verwendet, die im wesentlichen ähnlich wie bei Sampath et al. (PNAS 80 (1983), 6591-6595) beschrieben präpariert wurde. Die Rattenknochen (Femur und Tibia) wurden in 0,6 M HCL für 24 Stunden entmineralisiert und anschließend noch vorhandenes Knochenmark entfernt. Nach Waschen mit Wasser und dreistündigem Entfetten in einem Chloroform/Methanol (1/1) Gemisch wurden die Knochen luftgetrocknet, tiefgefroren in einer Mühle pulverisiert und Partikelgrößen zwischen 400 bis 1000 µm herausgesiebt. Anschließend wurde die Matrix für 7 Tage bei Raumtemperatur in 4 M Guanidinium-HCl in Gegenwart von Proteaseinhibitoren extrahiert. Nach extensivem Waschen mit Wasser wurde die Matrix lyophilisiert und bei 4°C aufbewahrt. So behandelte Matrices zeigen alleine keine knocheninduzierende Aktivität mehr.
Protein kann über verschiedene dem Fachmann bekannte Methoden mit der extrahierten Knochenmatrix kombiniert werden.
MP52-Protein bzw. Kontrollprotein, das sowohl über Heparin Sepharose als auch Reversed Phase HPLC gereinigt war, wurde in der Acetonitril/Trifluoressigsäure Lösung nach Elution mit je 25 mg Matrix pro Implantat vereinigt, gut gemischt, tiefgefroren und lyophilisiert. Für die Implantation von matrixgebundenem MP52 wurden zwei ca. 3 Monate alte Ratten (Whistar) verwendet, die durch intramuskuläre Injektion eines Narkotisierungsmittels (0,2 ml Rompun (Bayer) gemischt mit 0,5 ml Ketanest 50 (Parke Davis) ) mit 0,14 ml pro 100 g Körpergewicht betäubt worden waren. Für die Implantate wurden bilateral Taschen in der Bauchmuskulatur (unterhalb des Thorax, beginnend ca. 0,5 cm unterhalb des untersten Rippenbogens) präpariert. Das matrixgebundene MP52 (ca. 2 bis 4 µg nach Abschätzung auf Western blots) sowie die entsprechend matrixgebundenen Kontrollproteine wurden mit 0,9 %iger Kochsalzlösung (Delta Pharma) angefeuchtet und in die Muskeltaschen überführt. Die Muskeltaschen sowie die notwendigen Hautschnitte wurden anschließend vernäht. Die Ratten wurden mit Cyclosporin A (Sandimmun) immunsupprimiert.
Nach 18 bzw. nach 26 Tagen wurden die Implantate aus den Ratten entnommen und für histologische Untersuchungen fixiert. Da das Implantat mit MP52 nach 26 Tagen bereits makroskopisch die Bildung von Knochen vermuten ließ, wurde dieses zur Anfertigung von Dünnschnitten in Methylmethacrylat eingebettet, die anderen Implantate wurden in Paraffin eingebettet. Mineralisierte Knorpel- und Knochengewebe werden durch die von Kossa Färbetechnik (Romeis, B.; Mikroskopische Technik, Ed: Böck, P.; Urban und Schwarzenberg; München, Baltimore, Wien (1989)) schwarz hervorgehoben. Bei der Trichromfärbung nach Masson-Goldner (Romeis, B.; Mikroskopische Technik, Ed: Böck, P.; Urban und Schwarzenberg; München, Baltimore, Wien (1989)) wird mineralisiertes Knochengewebe und Kollagen leuchtend grün gefärbt, Osteoid ist rot und Zytoplasma rötlich-braun. Beide Färbetechniken wurden auf die Implantate aus beiden Ratten angewendet. Mit beiden Färbetechniken konnte in beiden Versuchstieren deutliche Knorpel- und Knochenbildung in den Implantaten, die MP52 enthalten, nachgewiesen werden. Die korrespondierenden Implantate mit Kontrollprotein zeigten keinerlei Knorpel- oder Knochenbildung. Der Anteil an Knorpelvorstufen mit Chondrozyten und Knorpelarealen mit beginnender Bildung von Extrazellulärmatrix und deren Mineralisierung in konzentrischen Kreisen ist in dem MP52 Implantat nach 18 Tagen höher als in dem von 26 Tagen. Aber auch in dem Implantat nach 18 Tagen sind bereits reifes Knochengewebe mit vektorieller Osteoidbildung sowie einzelne Osteocyten im Knochen nachweisbar. Weiterhin sind geschlossene Ossikel mit beginnender Knochenmarksbildung erkennbar. Bei dem Implantat nach 26 Tagen sind auch noch Knorpelareale mit beginnender Matrixbildung und Kalzifizierung nachweisbar, der Anteil an dem grün gefärbten mineralisierten Knochengewebe mit Osteocyten und Osteoidsäumen hat jedoch deutlich zugenommen. Auch in diesem Implantat ist Knochenmarksbildung mit vereinzelten Fettzellvorkommen nachweisbar. Zur Veranschaulichung zeigt Figur 5 den färberischen Nachweis (von Kossa) des Knochenmaterials vom Gesamtimplantat nach 26 Tagen. In Figur 6 ist ein kleiner Ausschnitt desselben Implantates nach Masson-Goldner Färbung gezeigt. Es zeigt aktiven Knochen mit einem Saum aus kubiodalen Osteoblasten und Osteoid in dem einzelne eingemauerte Osteoblasten erkennbar sind. Des weiteren sind einzelne Osteocyten im mineralisierten Knochengewebe (im Originalpräparat grün angefärbt) sichtbar. Die Knochenmarkbildung ist ebenfalls nachweisbar.

Der Versuch zeigt, daß rekombinant erzeugtes MP52 alleine, in Kombination mit einer Matrix, in der Lage ist enchondrale Knochenbildung zu induzieren.

### 2.2

Zur Bestätigung der Ergebnisse wurde ein weiterer ektopischer Knochenbildungstest unter Verwendung der MP52 L-Zelltransformanten durchgeführt. MP52 produzierende (Hind III-MP52/pABWN transfizierte) und nicht-produzierende (pABWN transfizierte) L-Zellen (1 x 10⁶ Zellen) wurden in die beidseitige Schenkelmuskulatur von je drei männlichen Nacktmäusen injiziert. Nach drei Wochen wurden alle Tiere getötet, die Schenkelmuskulatur abgetrennt und diese sowohl mit niedrigenergetischer Röntgenstrahlung als auch histopathologisch untersucht.

Wie in Tabelle 3 aufgelistet, zeigt die Röntgenstrahlanalyse dichtes Material an den Injektionsstellen im Muskelgewebe aller MP52-produzierenden L-Zellen. Mit histologischen Untersuchungen konnte einfache Knorpelbildung und kalzifizierte Knorpelbildung in den Muskeln festgestellt werden. Auch diese Ergebnisse bestätigen, daß MP52 enchondodrale Knochenbildung induzieren kann.

**Tabelle 3**

| | MP52 produzierende Zellen (HindIII-MP52/pABWN) | Kontrollzellen (pABWN) |
|---|---|---|
| dichtes Material bei Röntgenanalyse | 3/3 | 0/3 |
| Chondrocyten bei Histologie | 3/3 | 0/3 |
| Kalzifizierende Knorpelbildung bei Histologie | 3/3 | 0/3 |

Die durchgeführten Experimente bestätigen, daß MP52 Protein die Bildung von Knorpel aus undifferenzierten Mesenchymzellen, sowie die Differenzierung und Reifung von Osteoblasten stimuliert. Dies führt zu einer enchondralen Knochenbildung, die der Induktionskaskade bei der embryonalen Knochenbildung und der Knochenheilung bei Frakturen gleicht.

Die in den Versuchen aufgeführten Bedingungen sind als Illustration der MP52 Aktivität und nicht als Begrenzung zu betrachten. Die Erfindung kann auch in anderer Form untersucht und charakterisiert werden.

Für die in der Anmeldung genannten Zellinien und Plasmide sind als Anlage die Hinterlegungsbescheinigungen beigefügt, aus denen die Hinterlegungsangaben ersichtlich sind.

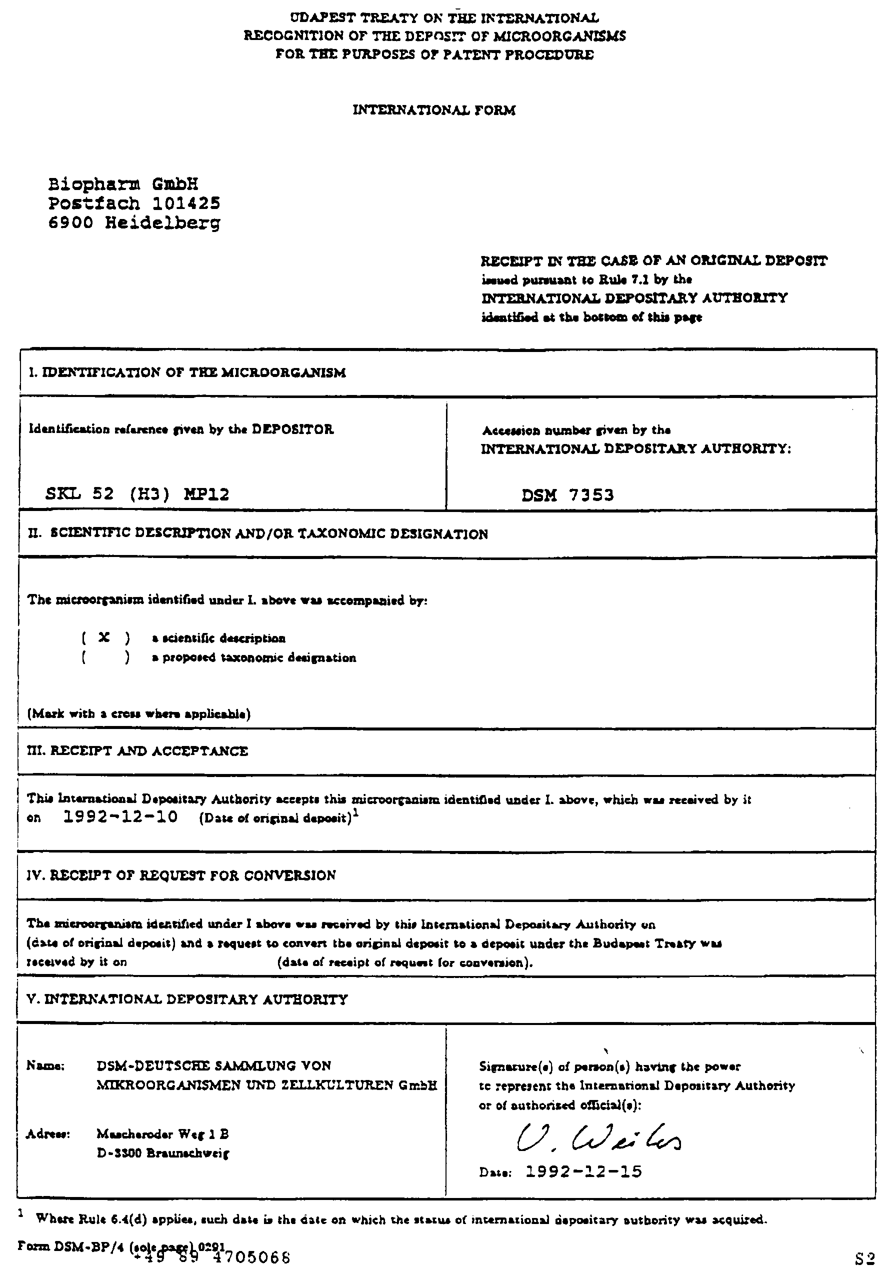
UDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Biopharm GmbH
Postfach 101425
6900 Heidelberg

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR | Accession number given by the
INTERNATIONAL DEPOSITARY AUTHORITY:

SKL 52 (H3) MP12 | DSM 7353

II. SCIENTIFIC DESCRIPTION AND/OR TAXONOMIC DESIGNATION

The microorganism identified under I. above was accompanied by:

( X ) a scientific description
( ) a proposed taxonomic designation

(Mark with a cross where applicable)

III. RECEIPT AND ACCEPTANCE

This International Depositary Authority accepts this microorganism identified under I. above, which was received by it
on 1992-12-10 (Date of original deposit)¹

IV. RECEIPT OF REQUEST FOR CONVERSION

The microorganism identified under I above was received by this International Depositary Authority on
(date of original deposit) and a request to convert the original deposit to a deposit under the Budapest Treaty was
received by it on (date of receipt of request for conversion).

V. INTERNATIONAL DEPOSITARY AUTHORITY

Name: DSM-DEUTSCHE SAMMLUNG VON
MIKROORGANISMEN UND ZELLKULTUREN GmbH | Signature(s) of person(s) having the power
to represent the International Depositary Authority
or of authorised official(s):

Adress: Mascheroder Weg 1 B
D-3300 Braunschweig |

Date: 1992-12-15

¹ Where Rule 6.4(d) applies, such date is the date on which the status of international depositary authority was acquired.

Form DSM-BP/4 (sole page) 0291
+49 89 4705068

S2

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Biopharm GmbH
Postfach 10 14 25
6900 Heidelberg

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR | Accession number given by the
INTERNATIONAL DEPOSITARY AUTHORITY:

Lambda 2.7.4 | DSM 7387

II. SCIENTIFIC DESCRIPTION AND/OR TAXONOMIC DESIGNATION

The microorganism identified under I. above was accompanied by:

( X ) a scientific description
( X ) a proposed taxonomic designation

(Mark with a cross where applicable)

III. RECEIPT AND ACCEPTANCE

This International Depositary Authority accepts this microorganism identified under I. above, which was received by it
on 1993-01-13 (Date of original deposit)¹

IV. RECEIPT OF REQUEST FOR CONVERSION

The microorganism identified under I above was received by this International Depositary Authority on
(date of original deposit) and a request to convert the original deposit to a deposit under the Budapest Treaty was
received by it on (date of receipt of request for conversion).

V. INTERNATIONAL DEPOSITARY AUTHORITY

Name: DSM-DEUTSCHE SAMMLUNG VON
MIKROORGANISMEN UND ZELLKULTUREN GmbH | Signature(s) of person(s) having the power
to represent the International Depositary Authority
or of authorized official(s):

Adress: Mascheroder Weg 1 B
D-3300 Braunschweig | U. Weihs

Date: 1993-01-15

¹ Where Rule 6.4(d) applies, such date is the date on which the status of international depositary authority was acquired.

Form DSM-BP/4 (sole page) 0[?]9[?] [?]70506S

S4

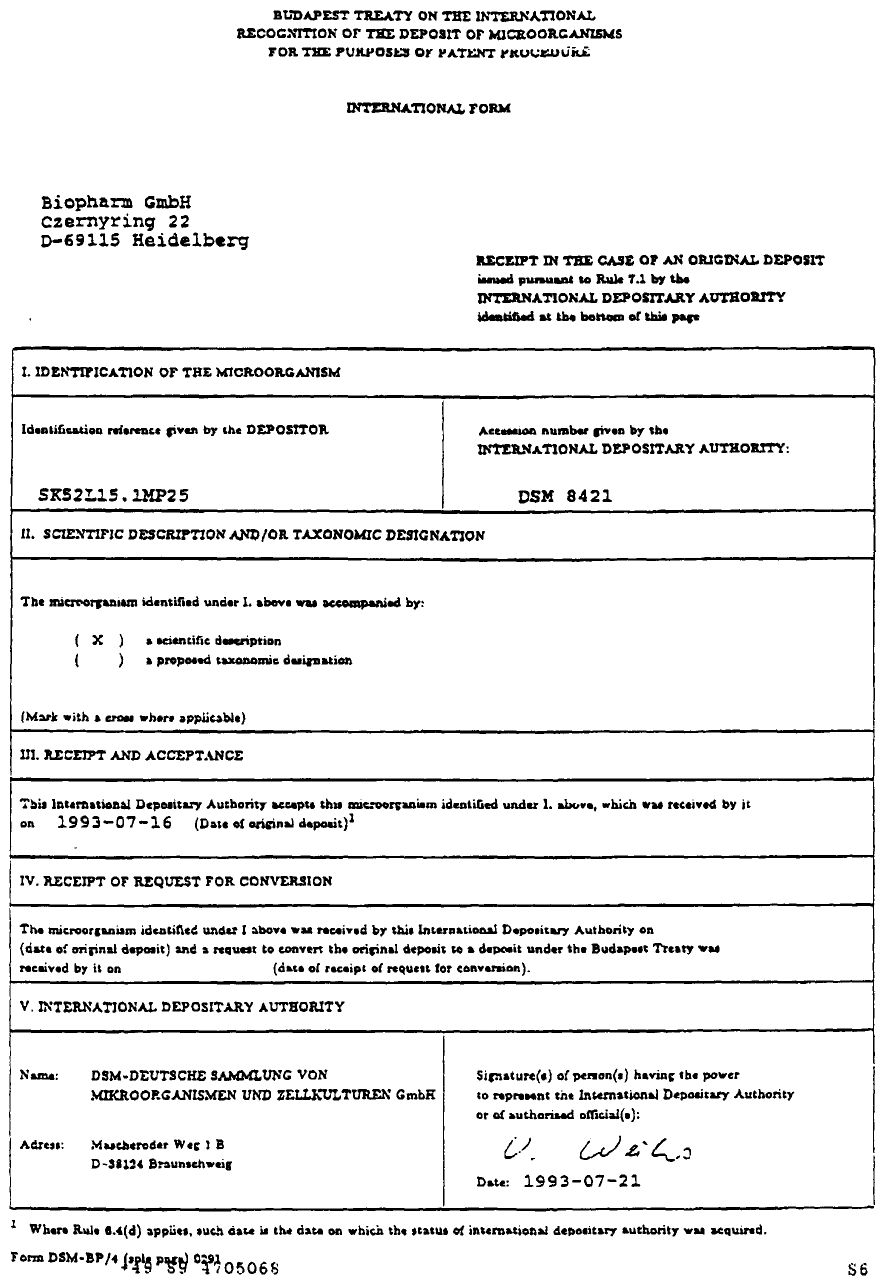
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Biopharm GmbH
Czernyring 22
D-69115 Heidelberg

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR | Accession number given by the
INTERNATIONAL DEPOSITARY AUTHORITY:

SK52L15.1MP25 | DSM 8421

II. SCIENTIFIC DESCRIPTION AND/OR TAXONOMIC DESIGNATION

The microorganism identified under I. above was accompanied by:

( X ) a scientific description
( ) a proposed taxonomic designation

(Mark with a cross where applicable)

III. RECEIPT AND ACCEPTANCE

This International Depositary Authority accepts the microorganism identified under I. above, which was received by it
on 1993-07-16 (Date of original deposit)¹

IV. RECEIPT OF REQUEST FOR CONVERSION

The microorganism identified under I above was received by this International Depositary Authority on
(date of original deposit) and a request to convert the original deposit to a deposit under the Budapest Treaty was
received by it on (date of receipt of request for conversion).

V. INTERNATIONAL DEPOSITARY AUTHORITY

Name: DSM-DEUTSCHE SAMMLUNG VON
MIKROORGANISMEN UND ZELLKULTUREN GmbH | Signature(s) of person(s) having the power
to represent the International Depositary Authority
or of authorized official(s):

Adress: Mascheroder Weg 1 B
D-38124 Braunschweig |
Date: 1993-07-21

¹ Where Rule 6.4(d) applies, such date is the date on which the status of international depositary authority was acquired.

Form DSM-BP/4 (sole page) 0291
+49 59 4705068

S6

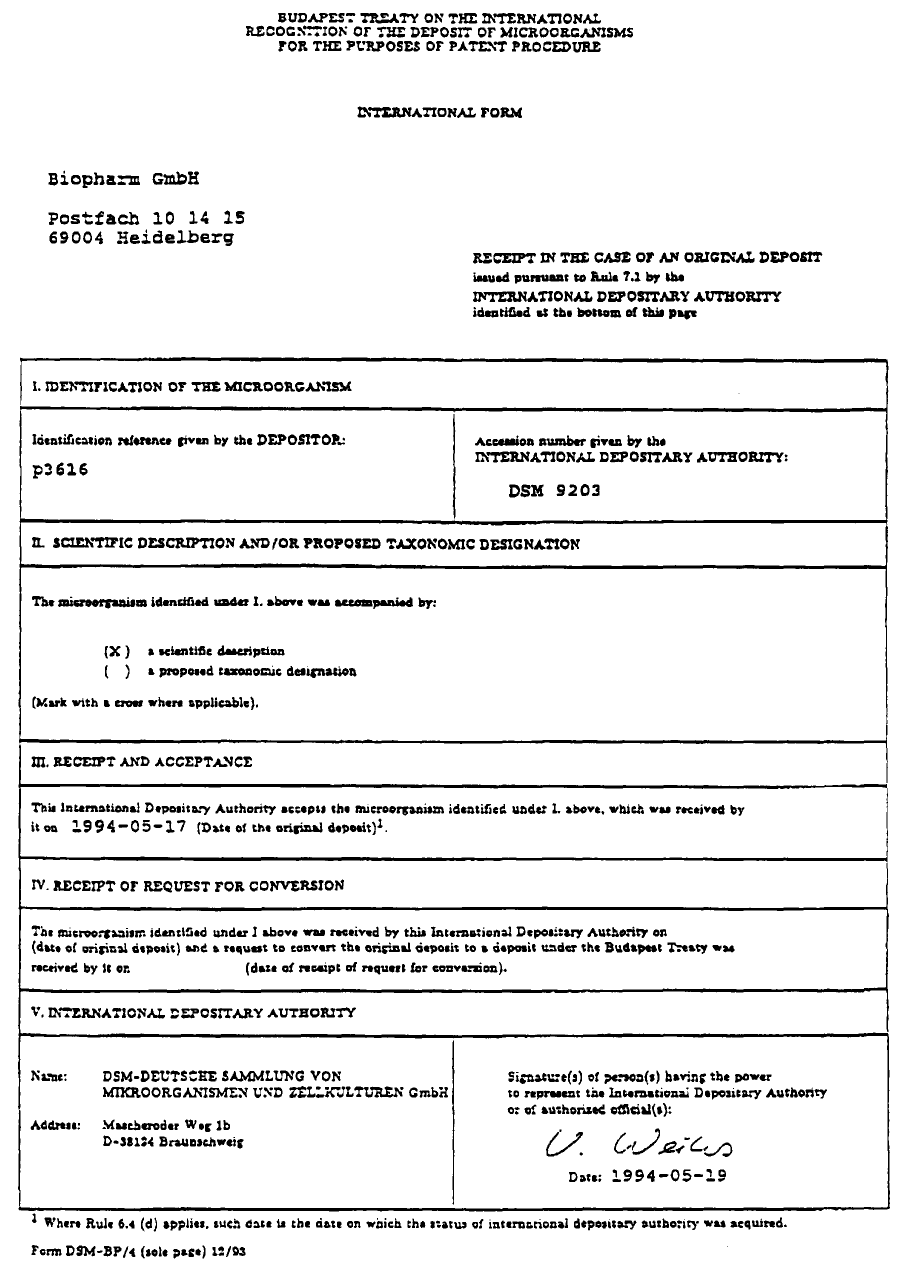
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Biopharm GmbH

Postfach 10 14 15
69004 Heidelberg

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR: | Accession number given by the
p3616 | INTERNATIONAL DEPOSITARY AUTHORITY:
 | DSM 9203

II. SCIENTIFIC DESCRIPTION AND/OR PROPOSED TAXONOMIC DESIGNATION

The microorganism identified under I. above was accompanied by:

(X) a scientific description
( ) a proposed taxonomic designation

(Mark with a cross where applicable).

III. RECEIPT AND ACCEPTANCE

This International Depositary Authority accepts the microorganism identified under I. above, which was received by
it on 1994-05-17 (Date of the original deposit)1.

IV. RECEIPT OF REQUEST FOR CONVERSION

The microorganism identified under I above was received by this International Depositary Authority on
(date of original deposit) and a request to convert the original deposit to a deposit under the Budapest Treaty was
received by it on (date of receipt of request for conversion).

V. INTERNATIONAL DEPOSITARY AUTHORITY

Name: DSM-DEUTSCHE SAMMLUNG VON | Signature(s) of person(s) having the power
MIKROORGANISMEN UND ZELLKULTUREN GmbH | to represent the International Depositary Authority
 | or of authorized official(s):
Address: Mascheroder Weg 1b |
D-38124 Braunschweig | Date: 1994-05-19

1 Where Rule 6.4 (d) applies, such date is the date on which the status of international depositary authority was acquired.

Form DSM-BP/4 (sole page) 12/93

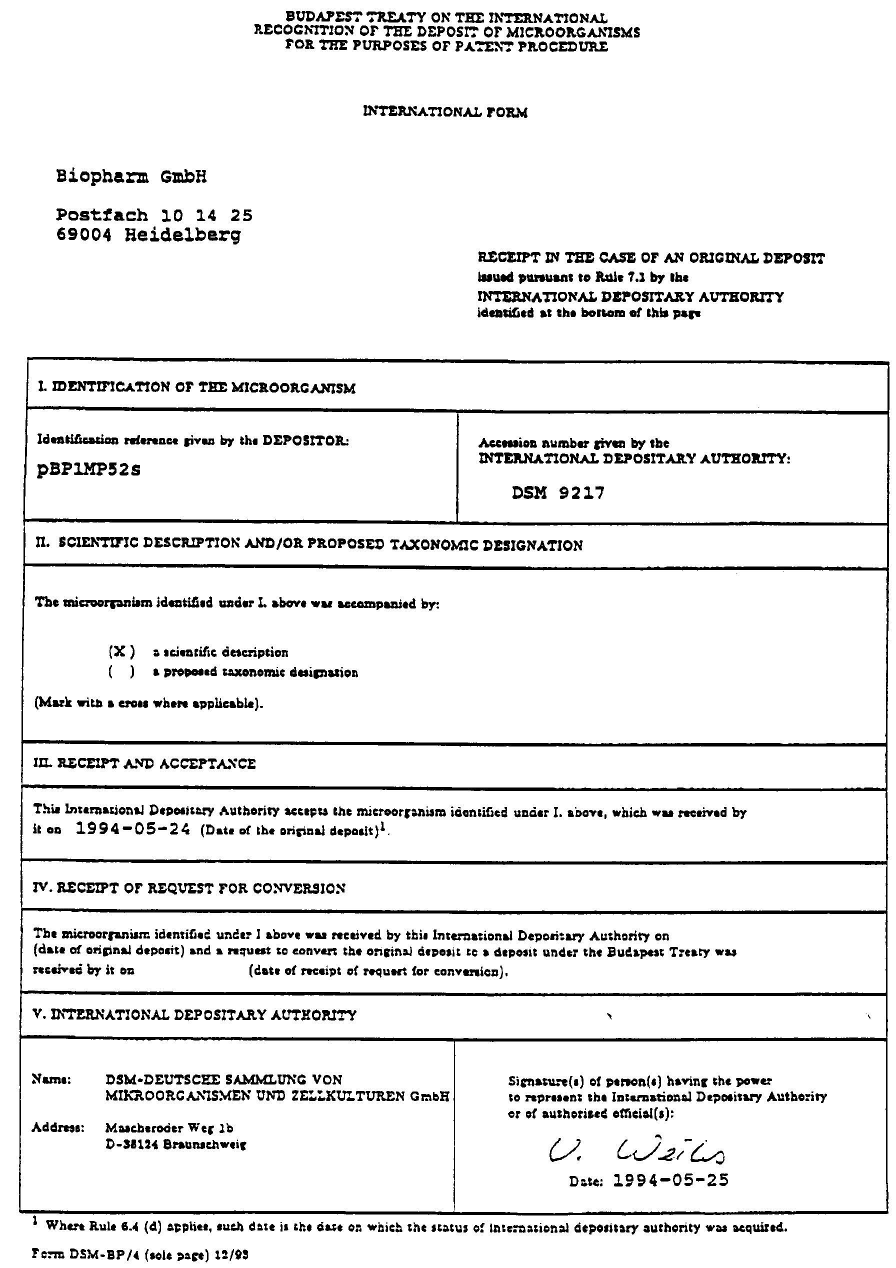
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Biopharm GmbH

Postfach 10 14 25
69004 Heidelberg

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR: | Accession number given by the
pBP1MP52s | INTERNATIONAL DEPOSITARY AUTHORITY:
 | DSM 9217

II. SCIENTIFIC DESCRIPTION AND/OR PROPOSED TAXONOMIC DESIGNATION

The microorganism identified under I. above was accompanied by:

(X) a scientific description
( ) a proposed taxonomic designation

(Mark with a cross where applicable).

III. RECEIPT AND ACCEPTANCE

This International Depositary Authority accepts the microorganism identified under I. above, which was received by
it on 1994-05-24 (Date of the original deposit)¹.

IV. RECEIPT OF REQUEST FOR CONVERSION

The microorganism identified under I above was received by this International Depositary Authority on
(date of original deposit) and a request to convert the original deposit to a deposit under the Budapest Treaty was
received by it on (date of receipt of request for conversion).

V. INTERNATIONAL DEPOSITARY AUTHORITY

Name: DSM-DEUTSCHE SAMMLUNG VON | Signature(s) of person(s) having the power
MIKROORGANISMEN UND ZELLKULTUREN GmbH | to represent the International Depositary Authority
 | or of authorised official(s):
Address: Mascheroder Weg 1b |
D-38124 Braunschweig |
 | Date: 1994-05-25

¹ Where Rule 6.4 (d) applies, such date is the date on which the status of international depositary authority was acquired.

Form DSM-BP/4 (sole page) 12/93

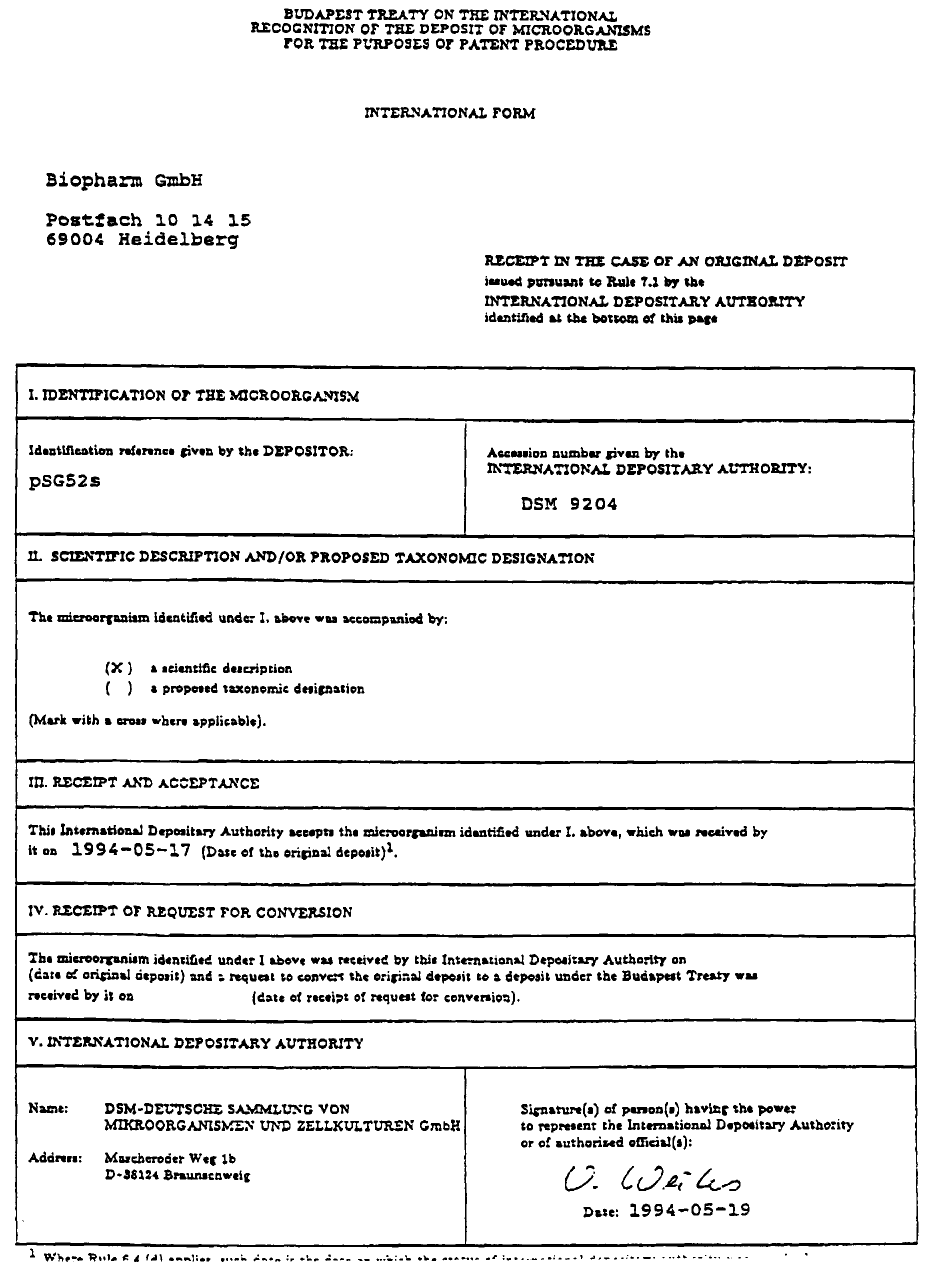
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Biopharm GmbH

Postfach 10 14 15
69004 Heidelberg

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR: | Accession number given by the INTERNATIONAL DEPOSITARY AUTHORITY:
pSG52s | DSM 9204

II. SCIENTIFIC DESCRIPTION AND/OR PROPOSED TAXONOMIC DESIGNATION

The microorganism identified under I. above was accompanied by:

(X) a scientific description
( ) a proposed taxonomic designation

(Mark with a cross where applicable).

III. RECEIPT AND ACCEPTANCE

This International Depositary Authority accepts the microorganism identified under I. above, which was received by
it on 1994-05-17 (Date of the original deposit)¹.

IV. RECEIPT OF REQUEST FOR CONVERSION

The microorganism identified under I above was received by this International Depositary Authority on
(date of original deposit) and a request to convert the original deposit to a deposit under the Budapest Treaty was
received by it on (date of receipt of request for conversion).

V. INTERNATIONAL DEPOSITARY AUTHORITY

Name: DSM-DEUTSCHE SAMMLUNG VON MIKROORGANISMEN UND ZELLKULTUREN GmbH | Signature(s) of person(s) having the power to represent the International Depositary Authority or of authorized official(s):
Address: Mascheroder Weg 1b D-38124 Braunschweig | V. Weihs
 | Date: 1994-05-19

¹ Where Rule 6.4 (d) applies, such date is the date on which the status of international depositary authority [?]

SEQ ID NO. 1
ART DER SEQUENZ: Nukleinsäuresequenz
NAME UND HERKUNFT: MP-52-DNA
LÄNGE: 2703 Nukleotide

CCATGGCCTCGAAAGGGCAGCGGTGATTTTTTTCACATAAATATATCGCACTTAAATGAG
TTTAGACAGCATGACATCAGAGAGTAATTAAATTGGTTTGGGTTGGAATTCCGTTTCCAA
TTCCTGAGTTCAGGTTTGTAAAAGATTTTTCTGAGCACCTGCAGGCCTGTGAGTGTGTGT
GTGTGTGTGTGTGTGTGTGTGTGTGTGTGAAGTATTTTCACTGGAAAGGATTCAAAACTA
GGGGGAAAAAAAAACTGGAGCACACAGGCAGCATTACGCCATTCTTCCTTCTTGGAAAAA
TCCCTCAGCCTTATACAAGCCTCCTTCAAGCCCTCAGTCAGTTGTGCAGGAGAAAGGGGG
CGGTTGGCTTTCTCCTTTCAAGAACGAGTTATTTTCAGCTGCTGACTGGAGACGGTGCAC
GTCTGGATACGAGAGCATTTCCACTATGGGACTGGATACAAACACACACCCGGCAGACTT
CAAGAGTCTCAGACTGAGGAGAAAGCCTTTCCTTCTGCTGCTACTGCTGCTGCCGCTGCT
TTTGAAAGTCCACTCCTTTCATGGTTTTTCCTGCCAAACCAGAGGCACCTTTGCTGCTGC
CGCTGTTCTCTTTGGTGTCATTCAGCGGCTGGCCAGAGGATGAGACTCCCCAAACTCCTC
ACTTTCTTGCTTTGGTACCTGGCTTGGCTGGACCTGGAATTCATCTGCACTGTGTTGGGT
GCCCCTGACTTGGGCCAGAGACCCCAGGGGACCAGGCCAGGATTGGCCAAAGCAGAGGCC
AAGGAGAGGCCCCCCCTGGCCCGGAACGTCTTCAGGCCAGGGGGTCACAGCTATGGTGGG
GGGGCCACCAATGCCAATGCCAGGGCAAAGGGAGGCACCGGGCAGACAGGAGGCCTGACA
CAGCCCAAGAAGGATGAACCCAAAAAGCTGCCCCCCAGACCGGGCGGCCCTGAACCCAAG
CCAGGACACCCTCCCCAAACAAGGCAGGCTACAGCCCGGACTGTGACCCCAAAAGGACAG
CTTCCCGGAGGCAAGGCACCCCCAAAAGCAGGATCTGTCCCCAGCTCCTTCCTGCTGAAG
AAGGCCAGGGAGCCCGGGCCCCCACGAGAGCCCAAGGAGCCGTTTCGCCCACCCCCCATC
ACACCCCACGAGTACATGCTCTCGCTGTACAGGACGCTGTCCGATGCTGACAGAAAGGGA
GGCAACAGCAGCGTGAAGTTGGAGGCTGGCCTGGCCAACACCATCACCAGCTTTATTGAC
AAAGGGCAAGATGACCGAGGTCCCGTGGTCAGGAAGCAGAGGTACGTGTTTGACATTAGT
GCCCTGGAGAAGGATGGGCTGCTGGGGGCCGAGCTGCGGATCTTGCGGAAGAAGCCCTCG
GACACGGCCAAGCCAGCGGCCCCCGGAGGCGGGCGGGCTGCCCAGCTGAAGCTGTCCAGC
TGCCCCAGCGGCCGGCAGCCGGCCTCCTTGCTGGATGTGCGCTCCGTGCCAGGCCTGGAC
GGATCTGGCTGGGAGGTGTTCGACATCTGGAAGCTCTTCCGAAACTTTAAGAACTCGGCC
CAGCTGTGCCTGGAGCTGGAGGCCTGGGAACGGGGCAGGGCCGTGGACCTCCGTGGCCTG
GGCTTCGACCGCGCCGCCCGGCAGGTCCACGAGAAGGCCCTGTTCCTGGTGTTTGGCCGC
ACCAAGAAACGGGACCTGTTCTTTAATGAGATTAAGGCCCGCTCTGGCCAGGACGATAAG

ACCGTGTATGAGTACCTGTTCAGCCAGCGGCGAAAACGGCGGCCCACTGGCCACTCGC
CAGGGCAAGCGACCCAGCAAGAACCTTAAGGCTCGCTGCAGTCGGAAGGCACTGCATGTC
AACTTCAAGGACATGGGCTGGGACGACTGGATCATCGCACCCCTTGAGTACGAGGCTTTC
CACTGCGAGGGGCTGTGCGAGTTCCCATTGCGCTCCCACCTGGAGCCCACGAATCATGCA
GTCATCCAGACCCTGATGAACTCCATGGACCCCGAGTCCACACCACCCACCTGCTGTGTG
CCCACGCGGCTGAGTCCCATCAGCATCCTCTTCATTGACTCTGCCAACAACGTGGTGTAT
AAGCAGTATGAGGACATGGTCGTGGAGTCGTGTGGCTGCAGGTAGCAGCACTGGCCCTCT
GTCTTCCTGGGTGGCACATCCCAAGAGCCCCTTCCTGCACTCCTGGAATCACAGAGGGGT
CAGGAAGCTGTGGCAGGAGCATCTACACAGCTTGGGTGAAAGGGGATTCCAATAAGCTTG
CTCGCTCTCTGAGTGTGACTTGGGCTAAAGGCCCCCTTTTATCCACAAGTTCCCCTGGCT
GAGGATTGCTGCCCGTCTGCTGATGTGACCAGTGGCAGGCACAGGTCCAGGGAGACAGAC
TCTGAATGGGACTGAGTCCCAGGAAACAGTGCTTTCCGATGAGACTCAGCCCACCATTTC
TCCTCACCTGGGCCTTCTCAGCCTCTGGACTCTCCTAAGCACCTCTCAGGAGAGCCACAG
GTGCCACTGCCTCCTCAAATCACATTTGTGCCTGGTGACTTCCTGTCCCTGGGACAGTTG
AGAAGCTGACTGGGCAAGAGTGGGAGAGAAGAGGAGAGGGCTTGGATAGAGTTGAGGAGT
GTGAGGCTGTTAGACTGTTAGATTTAAATGTATATTGATGAGATAAAAAGCAAAACTGTG
CCT

SEQ ID NO:2
ART DER SEQUENZ: Aminosäuresequenz
NAME UND HERKUNFT: MP-52-Protein
LÄNGE: 501 Aminosäuren

MRLPKLLTFL LWYLAWLDLE FICTVLGAPD LGQRPQGTRP GLAKAEAKER
PPLARNVFRP GGHSYGGGAT NANARAKGGT GQTGGLTQPK KDEPKKLPPR
PGGPEPKPGH PPQTRQATAR TVTPKGQLPG GKAPPKAGSV PSSFLLKKAR
EPGPPREPKE PFRPPPITPH EYMLSLYRTL SDADRKGGNS SVKLEAGLAN
TITSFIDKGQ DDRGPVVRKQ RYVFDISALE KDGLLGAELR ILRKKPSDTA
KPAAPGGGRA AQLKLSSCPS GRQPASLLDV RSVPGLDGSG WEVFDIWKLF
RNFKNSAQLC LELEAWERGR AVDLRGLGFD RAARQVHEKA LFLVFGRTKK
RDLFFNEIKA RSGQDDKTVY EYLFSQRRKR RAPLATRQGK RPSKNLKARC
SRKALHVNFK DMGWDDWIIA PLEYEAFHCE GLCEFPLRSH LEPTNHAVIQ
TLMNSMDPES TPPTCCVPTR LSPISILFID SANNVVYKQY EDMVVESCGC R

## Patentansprüche

1. DNA-Molekül, welches für ein Protein der TGF-β-Familie codiert und aus einer der folgenden DNA-Sequenzen besteht:
a) Nukleotiden 1783 - 2142 oder 640 bis 2142 der SEQ ID NO.1,
b) einer der Sequenzen aus a) im Rahmen der Degeneration des genetischen Codes entsprechende Nukleotidsequenz,
c) einem allelischen Derivat einer der Sequenzen aus a) und b) entsprechende Nukleotidsequenz, oder
d) einer mit einer der Sequenzen aus a), b) oder c) bei stringenten Hybridisierungsbedingungen hybridisierende Nukleotidsequenz unter der Voraussetzung, dass ein DNA-Molekül gemäß d) zumindest den für ein reifes Protein der TGF-β-Familie codierenden Anteil vollständig enthält, und die Nukleotidsequenz nicht zu SEQ ID NO.1 vollständig komplementär ist.

2. Vektor,
**dadurch gekennzeichnet,**
daß er mindestens eine Kopie eines DNA-Moleküls nach Anspruch 1 enthält.

3. Wirtszelle,
**dadurch gekennzeichnet,**
daß sie mit einer DNA nach Anspruch 1 oder einem Vektor nach Anspruch 2 transformiert ist.

4. Wirtszelle nach Anspruch 3,
**dadurch gekennzeichnet,**
daß sie ein Bakterium, ein Pilz, eine pflanzliche oder eine tierische Zelle ist.

5. Protein der TGF-β-Familie, das von einer DNA-Sequenz nach Anspruch 1 codiert wird.

6. Protein nach Anspruch 5,
**dadurch gekennzeichnet,**
daß es die in SEQ ID NO. 2 gezeigte Aminosäuresequenz oder gegebenenfalls funktionelle Anteile davon aufweist.

7. Verfahren zur Herstellung eines Proteins der TGF-β-Familie,
**dadurch gekennzeichnet,**
daß man eine Wirtszelle nach Anspruch 3 oder 4 kultiviert und das TGF-β-Protein aus der Zelle oder/und dem Kulturüberstand gewinnt.

8. Pharmazeutische Zusammensetzung,
**dadurch gekennzeichnet,**
daß sie mindestens ein Protein nach Anspruch 5 oder 6 als Wirkstoff gegebenenfalls zusammen mit pharmazeutisch üblichen Träger-, Hilfs-, Verdünnungs- oder Füllstoffen enthält.

9. Pharmazeutische Zusammensetzung nach Anspruch 8,
**dadurch gekennzeichnet,**
daß der Wirkstoff auf ein natürliches oder synthetisches Matrixmaterial aufgebracht und/oder darin integriert ist.

10. Pharmazeutische Zusammensetzung nach Anspruch 9,
**dadurch gekennzeichnet,**
daß das Matrixmaterial ein biokompatibles, in vivo biologisch abbaubares poröses Material ist.

11. Pharmazeutische Zusammensetzung nach einem der Ansprüche 8 bis 10 zur Behandlung oder Prävention von Knochen-, Knorpel-, Bindegewebs-, Haut-, Schleimhaut-, Epithelial- oder Zahnschädigungen, zur Anwendung bei Zahnimplantaten und zur Anwendung in Wundheilungs- und Gewebewiederherstellungsprozessen.

12. Antikörper oder Antikörperfragmente,
**dadurch gekennzeichnet,**
daß sie an ein Protein nach Anspruch 5 oder 6 binden.

## Claims

1. DNA molecule that codes for a protein of the TGF-β family and is composed of one of the following DNA sequences:
(a) nucleotides 1783 - 2142 or 640 to 2142 of SEQ ID NO.1,
(b) a nucleotide sequence corresponding to a sequence from a) within the scope of the degeneracy of the genetic code,
(c) an allelic derivative of a nucleotide sequence corresponding to one of the sequences from a) and b) or
(d) a nucleotide sequence hybridizing under stringent hybridization conditions with one of the sequences from a), b) or c) provided that a DNA molecule according to d) contains at least the part coding for a mature protein of the TGF-β family and the nucleotide sequence is not completely complementary to SEQ ID NO.1.

2. Vector,
**wherein**
it contains at least one copy of a DNA molecule as claimed in claim 1.

3. Host cell,
**wherein**
it is transformed by a DNA as claimed in claim 1 or by a vector as claimed in claim 2.

4. Host cell as claimed in claim 3,
**wherein**
it is a bacterium, a fungus, a plant or an animal cell.

5. Protein of the TGF-β family which is coded by a DNA sequence as claimed in claim 1.

6. Protein as claimed in claim 5,
**wherein**
it has the amino acid sequence shown in SEQ ID NO. 2 or optionally functional parts thereof.

7. Process for the production of a protein of the TGF-β family,
**wherein**
a host cell as claimed in claim 3 or 4 is cultured and the TGF-β protein is isolated from the cell or/and from the culture supernatant.

8. Pharmaceutical composition,
**wherein**
it contains at least one protein as claimed in claim 5 or 6 as the active substance optionally together with the usual pharmaceutical carrier substances, auxiliary substances, diluents or fillers.

9. Pharmaceutical composition as claimed in claim 8,
**wherein**
the active substance is applied to and/or integrated into a natural or synthetic matrix material.

10. Pharmaceutical composition as claimed in claim 9,
**wherein**
the matrix material is a biocompatible, porous material that can be biologically degraded in vivo.

11. Pharmaceutical composition as claimed in one of the claims 8 to 10 for the treatment or prevention of damage to bone, cartilage, connective tissues, skin, mucous membranes, epithelium or teeth, for application in dental implants and for application in wound-healing and tissue regeneration processes.

12. Antibodies or antibody fragments,
**wherein**
they bind to a protein as claimed in claim 5 or 6.

## Revendications

1. Molécule d'ADN qui code une protéine de la famille de TGF-β et qui consiste en l'une des séquences d'ADN suivantes:
a) nucléotides 1783 - 2142 ou 640 à 2142 de SEQ ID NO.1,
b) une séquence nucléotidique correspondant aux séquences de a) dans le cadre de la dégénérescence du code génétique,
c) un dérivé allélique d'une séquence nucléotidique correspondant aux séquences de a) et b), ou
d) une séquence nucléotidique qui s'hybride avec l'une des séquences de a), b) ou c) dans des conditions d'hybridation stringentes à condition qu'une molécule d'ADN selon d) contienne totalement au moins la partie codant une protéine mature de la famille de TGF-β et que la séquence nucléotidique ne soit pas totalement complémentaire de SEQ ID NO.1.

2. Vecteur caractérisé en ce qu'il contient au moins une copie d'une molécule d'ADN selon la revendication 1.

3. Cellule-hôte caractérisée en ce qu'elle est transformée avec un ADN selon la revendication 1 ou un vecteur selon la revendication 2.

4. Cellule-hôte selon la revendication 3 caractérisée en ce que c'est une bactérie, un champignon, une cellule végétale ou une cellule animale.

5. Protéine de la famille de TGF-β qui est codée par une séquence d'ADN selon la revendication 1.

6. Protéine selon la revendication 5 caractérisée en ce qu'elle présente la séquence d'acides aminés montrée dans SEQ ID NO.2 ou éventuellement des parties fonctionnelles de celle-ci.

7. Procédé de production d'une protéine de la famille de TGF-β caractérisé en ce que l'on cultive une cellule-hôte selon la revendication 3 ou 4 et on obtient la protéine TGF-β à partir de la cellule et/ou du surnageant de culture.

8. Composition pharmaceutique caractérisée en ce qu'elle contient au moins une protéine selon la revendication 5 ou 6 comme principe actif éventuellement en même temps que des supports, adjuvants, diluants ou charges pharmaceutiquement habituels.

9. Composition pharmaceutique selon la revendication 8 caractérisée en ce que le principe actif est appliqué sur et/ou intégré dans un matériau formant matrice naturel ou synthétique.

10. Composition pharmaceutique selon la revendication 9 caractérisée en ce que le matériau formant matrice est un matériau poreux biocompatible, biodégradable in vivo.

11. Composition pharmaceutique selon l'une des revendications 8 à 10 pour le traitement ou la prévention des lésions osseuses, des cartilages, du tissu conjonctif, de la peau, des muqueuses, épithéliales ou dentaires, pour l'application dans les implants dentaires et pour l'application dans les processus de cicatrisation des plaies et de restauration des tissus.

12. Anticorps ou fragments d'anticorps caractérisés en ce qu'ils se lient à une protéine selon la revendication 5 ou 6.
